# FASCICULE DE BREVET EUROPEEN

(11) **EP 1 038 863 B1**
(45) Date de publication et mention de la délivrance du brevet: **04.06.2003**
(21) Numéro de dépôt: 00400813.2
(22) Date de dépôt: 24.03.2000
(51) Int. Cl.: C07C 233/60, A61K 31/16, A61K 31/33, A61P 5/00, C07C 233/18, C07C 275/24, C07C 233/20, C07C 233/73, C07C 235/34, C07C 233/36, C07D 307/79, C07D 311/58, C07D 333/58, C07D 307/81, C07D 209/16, C07D 319/20, C07D 519/00, C07C 323/42, C07C 271/16, C07D 307/68

(54) **Nouveaux dérivés dimériques substitués, leur procédé de préparation et les compositions pharmaceutiques qui les contiennent**
Substituierte dimere Verbindungen Verfahren zu deren Herstellung und deren pharmazeutischen Zusammensetzungen
Substituted dimeric compounds, process for their preparation and pharmaceutical compositions thereof

(30) Priorité: 25.03.1999 FR 9903717
(43) Date de publication de la demande: 27.09.2000
(73) Titulaire: LES LABORATOIRES SERVIER, 92200 Neuilly sur Seine (FR)
(72) Inventeur: Lesieur, Daniel, 59147 Gondecourt (FR); Yous, Said, 59000 Lille (FR); Descamps-Francois, Carole, 59260 Hellemmes (FR); Lefoulon, Francois, 45000 Orleans (FR); Guillaumet, Gérald, 45650 Saint Jean Le Blanc (FR); Viaud, Marie-Claude, 37000 Tours (FR); Bennejean, Caroline, 94220 Charenton le Pont (FR); Delagrange, Philippe, 92130 Issy les Moulineaux (FR); Renard, Pierre, 78150 Le Chesnay (FR)

(56) Documents cités:
- US-A- 5 591 775
- CHEMICAL ABSTRACTS, vol. 99, no. 11, 12 septembre 1983 (1983-09-12) Columbus, Ohio, US; abstract no. 87984, SAMSONIYA, SH. A. ET AL: "Some alkylation reactions of bis[(dimethylamino)methyl] derivatives of di-5-indolylmethane" XP002125388 & SOOBSHCH. AKAD. NAUK GRUZ. SSR (1983), 109(1), 73-6, 1983,
- CHEMICAL ABSTRACTS, vol. 99, no. 1, 4 juillet 1983 (1983-07-04) Columbus, Ohio, US; abstract no. 5476, SAMSONIYA, SH. A. ET AL: "Some reactions of bis(cyanomethyl) derivatives of bis(5- indolyl)methane" XP002125389 & SOOBSHCH. AKAD. NAUK GRUZ. SSR (1982), 108(3), 561-3, 1982,
- LI P -K ET AL: "The development of a charged melatonin receptor ligand" BIOORGANIC & MEDICINAL CHEMISTRY LETTERS,GB,OXFORD, vol. 7, no. 18, page 2409-2414 XP004136454 ISSN: 0960-894X
- M. MOR ET AL.: "Melatonin receptor ligands: Synthesis of new melatonin derivatives and comprehensive comparative moleculatr field analysis (CoMFA) study" JOURNAL OF MEDICINAL CHEMISTRY, vol. 41, 1998, pages 3831-3844, XP002125401 WASHINGTON US

## Description

La présente invention concerne de nouveaux dérivés dimériques substitués, leur procédé de préparation et les compositions pharmaceutiques qui les contiennent.

On connaît dans l'art antérieur des structures dimériques en série naphtalénique (J. Chem. Soc., Dalton Trans., 1979. (10), pp. 1497-502) étudiées pour leurs propriétés de coordination au sein de complexes métalliques. Des dimères d'indoles sont par ailleurs décrits pour leur activité « curare-like » (Khim.-Farm. Zh., 1984, 18 (1), pp. 29-31).

Les composés de la présente invention, de par leur structure originale, sont nouveaux et présentent des propriétés pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.

Outre leur action bénéfique sur les troubles du rythme circadien (J. Neurosurg. 1985, 63, pp. 321-341) et du sommeil (Psychopharmacology, 1990, 100, pp. 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp. 264-272) et analgésiques (Pharmacopsychiat., 1987, 20, pp. 222-223) ainsi que pour le traitement des maladies de Parkinson (J. Neurosurg. 1985, 63, pp. 321-341) et d'Alzheimer (Brain Research, 1990, 528, pp. 170-174). De même, ces composés ont montré une activité sur certains cancers (Melatonin - Clinical Perspectives, Oxford University Press, 1988, pp. 164-165), sur l'ovulation (Science 1987, 227, pp. 714-720), sur le diabète (Clinical Endocrinology, 1986. 24, pp. 359-364), et dans le traitement de l'obésité (International Journal of Eating Disorders, 1996, 20 (4), pp. 443-446).

Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sous-types réceptoriels mélatoninergiques.

La présente invention concerne plus particulièrement les composés de formule (I) :

A-G₁-Cy-G₂-Cy-G₃-B (I)

dans laquelle :
◆ A représente un groupement de formule 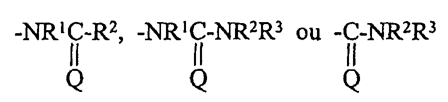 dans lesquelles :
   - Q représente un atome de soufre ou d'oxygène,
   - R¹, R², R³, identiques ou différents, représentent un atome d'hydrogène ou un groupement Rₐ [où Rₐ représente un groupement alkyle (C₁-C₆) linéaire ou ramifié non substitué ou substitué, alkényle (C₂-C₆) linéaire ou ramifié non substitué ou substitué, alkynyle (C₂-C₆) linéaire ou ramifié non substitué ou substitué, cycloalkyle (C₃-C₈) non substitué ou substitué, cycloalkyl (C₃-C₈) alkyle (C₁-C₆) linéaire ou ramifié non substitué ou substitué, polyhalogénoalkyle (C₁-C₆) linéaire ou ramifié, aryle, arylalkyle (dans lequel la partie alkyle contient 1 à 6 atomes de carbone et peut être linéaire ou ramifiée), arylalkényle (dans lequel la partie alkényle contient 2 à 6 atomes de carbone et peut être linéaire ou ramifiée), hétéroaryle, hétéroarylalkyle (dans lequel la partie alkyle contient 1 à 6 atomes de carbone et peut être linéaire ou ramifiée) ou hétéroarylalkényle (dans lequel la partie alkényle contient 2 à 6 atomes de carbone et peut être linéaire ou ramifiée)],
      ou les groupements R² et R³ forment, avec l'atome d'azote qui les porte un groupement choisi parmi piperazinyle, pipéridinyle, ou pyrrolidinyle,

   ◆ B représente un groupement de formule 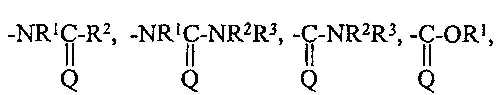 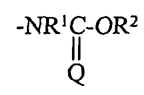 ou -NR²R³ où Q, R¹, R² et R³ sont tels que définis précédemment,
   ◆ G₁ et G₃, identiques ou différents, représentent une chaîne alkylène contenant de 1 à 4 carbones linéaire ou ramifiée éventuellement substituée par un ou plusieurs groupements, identiques ou différents, choisis parmi hydroxy, carboxy, formyle, Rₐ, ORₐ, COORₐ ou CORₐ (où Rₐ est tel que défini précédemment),
   ◆ Cy représente
      - une structure cyclique de formule (II) : 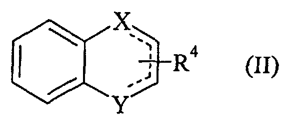
      dans laquelle :
      ∗ X et Y, identiques ou différents, représentent un atome de soufre, d'oxygène ou de carbone, ou un groupement CH ou CH₂,
      ∗ R⁴ représente un atome d'hydrogène ou d'halogène ou un groupement CF₃, hydroxy, carboxy, formyle, amino, NHRₐ, NRₐR¹ₐ, NHCORₐ, CONHRₐ, Rₐ, ORₐ, CORₐ ou COORₐ (où Rₐ est tel que défini précédemment et R¹ₐ peut prendre toutes les valeurs de Rₐ),
      ∗ la représentation ---- signifie que les liaisons sont simples ou doubles, étant entendu que la valence des atomes est respectée,
      où G₂ substitue le cycle benzénique, et G₁ (G₃ respectivement) substitue le cycle contenant X et Y,

   - ou une structure cyclique de formule (III) : 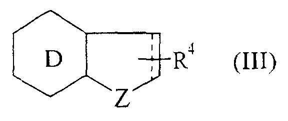
   dans laquelle :
   ∗ Z représente un atome de soufre ou d'oxygène, ou un groupement CH, CH₂, NH, NSO₂Ph ou NRₐ (où Rₐ est tel que défini précédemment),
   ∗ D représente un cycle benzénique ou pyridinique,
   ∗ R⁴ est tel que défini précédemment,
   ∗ la représentation ---- signifie que la liaison est simple ou double, étant entendu que la valence des atomes est respectée,
   où G₂ substitue le cycle D, et G₁ (G₃ respectivement) substitue le cycle contenant Z,
étant entendu que les deux cycles (Cy) des composés de formule (I) représentent la même structure cyclique de base (indole/indole, naphtalène/naphtalène, benzofurane/ benzofurane etc...), mais le substituant R⁴ peut être différent,
◆ G₂ représente une chaîne de formule (IV) 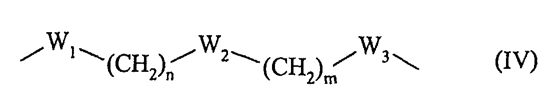 dans laquelle :
   - W₁, W₂ et W₃, identiques ou différents, représentent une liaison, un atome d'oxygène ou de soufre, ou un groupement CH₂, CHRₐ, NH ou NRₐ (où Rₐ est tel que défini précédemment),
   - n représente un entier tel que 0 ≤ n ≤ 6,
   - m représente un entier tel que 0 ≤ m ≤ 6,
étant entendu que l'on ne peut pas avoir deux hétéroatomes consécutifs, et que la chaîne de formule (IV) ainsi définie peut comporter une ou plusieurs insaturations,
étant entendu que :
- le composé de formule (I) ne peut représenter le 2-(acétylamino)-2-{[5-({3-[2-(acétylamino)-3-éthoxy-2-(éthoxycarbonyl)-3-oxopropyl]-*1H*-indol-5-yl}méthyl)-*1H*-indol-3-yl]méthyl}malonate de diéthyle, le 2-(acétylamino)-2-{[5-{[3-[2-(acétylamino)-3-éthoxy-2-(éthoxycarbonyl)-3-oxopropyl]-2-(éthoxycarbonyl)-*1H*-indol-5-yl]méthyl}-2-(éthoxycarbonyl)-1*H*-indol-3-yl]méthyl}malonate dediéthyle, ni le N-{2-[5-({3-[2-(acétylamino)éthyl]-*1H*-indol-5-yl}méthyl)-*1H*-indol-3-yl]éthyl} acétamide,
- par « aryle », on entend les groupements naphtyle, phényle et biphényle,
- par « hétéroaryle », on entend tout groupement mono ou bicyclique, saturé ou insaturé contenant 5 à 10 atomes et contenant 1 à 3 hétéroatomes choisis parmi azote, soufre et oxygène,
   les groupements « aryles » et « hétéroaryles » pouvant être substitués par un ou plusieurs radicaux, identiques ou différents, choisis parmi hydroxy, carboxy, alkoxy (C₁-C₆) linéaire ou ramifié, alkyle (C₁-C₆) linéaire ou ramifié, polyhalogénoalkyle(C₁-C₆) linéaire ou ramifié, formyle, cyano, nitro, amino, alkylamino (C₁-C₆) linéaire ou ramifié, dialkylamino (C₁-C₆) linéaire ou ramifié, ou atomes d'halogène,
- le terme « substitué » affecté aux expressions « alkyle », « «alkényle», et «alkynyle» signifie que ces groupements sont substitués par un ou plusieurs radicaux, identiques ou différents, choisis parmi hydroxy, alkoxy (C₁-C₆) linéaire ou ramifié, polyhalogénoalkyle (C₁-C₆) linéaire ou ramifié, amino, alkylamino (C₁-C₆) linéaire ou ramifié, dialkylamino (C₁-C₆) linéaire ou ramifié, ou atomes d'halogène,
- le terme « substitué » affecté aux expressions « cycloalkyle » et « cycloalkylalkyle » signifie que la partie cyclique de ces groupements est substituée par un ou plusieurs radicaux, identiques ou différents, choisis parmi hydroxy, alkoxy (C₁-C₆) linéaire ou ramifié, polyhalogénoalkyle (C₁-C₆) linéaire ou ramifié, amino, alkylamino (C₁-C₆) linéaire ou ramifié, dialkylamino (C₁-C₆) linéaire ou ramifié, ou atomes d'halogène,
leurs énantiomères et diastéréoisomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I) pour lesquels Cy représente une structure cyclique de formule (II) comme le naphtalène ou le tétrahydronaphtalène par exemple, ou de formule (III) comme l'indole, l'azaindole, le benzothiophène ou le benzofurane par exemple.

De façon avantageuse, l'invention concerne les composés de formule (I) pour lesquels G₂ représente une liaison simple,
ou un groupement -W₄-(CH₂)ₚ-W'₄- (où W₄ et W'₄, identiques ou différents, représentent un atome d'oxygène ou de soufre, ou un groupement NH ou NRₐ, et p représente un entier tel que 1 ≤ p ≤ 12) comme par exemple le groupement -O-(CH₂)ₚ-O- (où p est tel que défini précédemment),
ou un groupement de formule -W₄-(CH₂)_{p'}-W'₄-(CH₂)_{p"}-W"₄- (où W₄, W'₄ et W"₄, identiques ou différents, représentent un atome d'oxygène ou de soufre, ou un groupement NH ou NRₐ, et p' et p" sont deux entiers tels que 2 ≤ p' + p" ≤ 12) comme par exemple le groupement -O-(CH₂)_{p'}-O-(CH₂)_{p"}-O- (où p' et p" sont tels que définis précédemment).

Les substituants A et B préférés de l'invention sont les groupements NR¹C(Q)R², NR¹C(Q)NR²R³ ou C(Q)NR²R³, et plus particulièrement les groupements NR¹COR² ou CONR²R³.

Encore plus particulièrement, l'invention concerne les composés de formule (I) qui sont :
- le *N*-(2-{7-[2-({8-[2-(acétylamino)éthyl]-2-naphtyl}oxy)éthoxy]-1-naphtyl}éthyl) acétamide,
- le *N*-(2-{7-[3-({8-[2-(acétylamino)éthyl]-2-naphtyl}oxy)propoxy]-1-naphtyl} éthyl)acétamide,
- le *N*-(2-{7-[4-({8-[2-(acétylamino)éthyl]-2-naphtyl}oxy)butoxy]-1-naphtyl}éthyl) acétamide,
- le *N*-[2-(7-{[6-({8-[2-(acétylamino)éthyl]-2-naphtyl}oxy)hexyl]oxy}-1-naphtyl) éthyl]acétamide,
- le *N*-[2-(7-{[8-({8-[2-(acétylamino)éthyl]-2-naphtyl}oxy)octyl]oxy}-1-naphtyl) éthyl]acétamide,
- le *N*-[2-(7-{[10-({8-[2-(acétylamino)éthyl]-2-naphtyl}oxy)décyl]oxy}-1-naphtyl) éthyl]acétamide,
- le *N*-[2-(7-{[5-({8-[2-(acétylamino)éthyl]-2-naphtyl}oxy)pentyl]oxy}-1-naphtyl) éthyl]acétamide,
- le *N*-(2-{7-[4-({8-[2-(2-furoylamino)éthyl]-2-naphtyl}oxy)butoxy]-1-naphtyl} éthyl)-2-furamide,
- le 2-bromo-*N*-[2-(7-{4-[(8-{2-[(bromoacétyl)amino]éthyl}-2-naphtyl)oxy] butoxy}-1-naphtyl)éthyl]acétamide,
- le *N*-[2-(7-{4-[(8-{2-[(cyclopropylcarbonyl)amino]éthyl}-2-naphtyl)oxy] butoxy}-1-naphtyl)éthyl]cyclopropane carboxamide,
- le *N*-(2-{7-[4-({8-[2-(3-buténoylamino)éthyl]-2-naphtyl}oxy)butoxy]-1-naphtyl} éthyl)-3-buténamide,
- le *N*-(2-{7-[4-({8-[2-(acétylamino)éthyl]-7-méthoxy-2-naphtyl}oxy)butoxy]-2-méthoxy-1-naphtyl}éthyl)acétamide,
- le *N*-[2-(7-{2-[2-({8-[2-(acétylamino)éthyl]-2-naphtyl}oxy)éthoxy]éthoxy}-1-naphtyl)éthyl]acétamide,
- le 2-{7-[4-({8-[2-(acétylamino)éthyl]-2-naphtyl]}oxy)butoxy]-1-naphtyl}éthyl carbamate de *tert*-butyle,
- le *N*-{2-[7-(4-{{8-(2-aminoéthyl)-2-naphtyl]oxy}butoxy)-1-naphtyl]éthyl} acétamide, chlorhydrate,
- le {7-[4-({8-[2-(acétylamino)éthyl]-2-naphtyl}oxy)butoxy]-1-naphtyl}acétate de méthyle,
- l'acide {7-[4-({8-[2-(acétylamino)éthyl]-2-naphtyl}oxy)butoxy]-1-naphtyl} acétique,
- le *N*-(2-{7-[4-({8-[2-(acétylamino)éthyl]-5,6,7,8-tétrahydro-2-naphtalényl}oxy) butoxy]-1,2,3,4-tétrahydro-1-naphtalényl}éthyl)acétamide,
- le *N*-{2-[5-(4-{[3-[2-(acétylamino)éthyl]-1-(phénylsulfonyl)-*1H*-indol-5-yl] oxy}butoxy)-1-(phénylsulfonyl)-*1H*-indol-3-yl]éthyl}acétamide,
- le *N*-(2-{5-[4-({3-[2-(acétylamino)éthyl)-*1H*-indol-5-yl}oxy)butoxy]-*1H*-indol-3-yl}éthyl)acétamide,
- le *N*-(2-{5-[4-({3-[2-(acétylamino)éthyl]-1-benzofuran-5-yl}oxy)butoxy]-1-benzofuran-3-yl}éthyl)acétamide,
- le *N*-(2-{5-[4-({3-[2-(acétylamino)ethyl]-1-benzothién-5-yl}oxy)butoxy]-1-benzothién-3-yl}éthyl)acétamide,
- le *N*-(2-{5-[4-({3-[2-(acétylamino)éthyl]-1-méthyl-*1H*-pyrrolo*[3,2-b]*pyridin-5-yl}oxy)butoxy]-1-méthyl-*1H*-pyrrolo*[3,2-b]*pyridin-3-yl}éthyl)acétamide,
- le *N*-(2-{5-[4-({3-[2-(acétylamino)éthyl]-1-méthyl-*1H*-pyrrolo*[3,2-b]*pyridin-5-yl}oxy)propoxy]-1-méthyl-*1H*-pyrrolo*[3,2-b]*pyridin-3-yl}éthyl)acétamide,
- le *N*-[2-(7-{8-[2-(acétylamino)éthyl]-2-naphtyl}-1-naphtyl)éthyl]acétamide,
- le *N*-{2-[5-{3-[2-(acétylamino)éthyl]-*1H*-indol-3-yl}-*1H*-indol-3-yl]éthyl}acétamide.

Les énantiomères, diastéréoisomères ainsi que les sels d'addition à un acide ou une base pharmaceutiquement acceptable des composés préférés de l'invention font partie intégrante de l'invention.

La présente invention concerne également le procédé de préparation des composés de formule (I) caractérisé en ce que l'on utilise comme produit de départ le composé de formule (V) :

A-G₁-Cy-OMe (V)

dans laquelle A, G₁ et Cy sont tels que définis dans la formule (I),
que l'on soumet à une déméthylation en utilisant des agents classiques comme HBr, AlCl₃, AlBr₃, BBr₃ ou des systèmes binaires acide de Lewis / nucléophile comme AlCl₃ / PhCH₂SH ou BBr₃ / Me₂S par exemple, pour obtenir le composé de formule (VI) :

A-G₁-Cy-OH (VI)

dans laquelle A, G₁ et Cy sont définis comme précédemment.
◆ qui est transformé, de façon classique,
   - par action de *N,N*-diméthylthiocarbamate de sodium par exemple, en thiol correspondant de formule (VII) :

      A-G₁-Cy-SH (VII)

      dans laquelle A, G₁ et Cy sont tels que définis précédemment,
   - ou en dérivé aminé correspondant de formule (VIII) :

      A-G₁-Cy-NHR'ₐ (VIII)

      dans laquelle A, G₁ et Cy sont définis comme précédemment et R'ₐ peut prendre toutes les valeurs de Rₐ tel que défini dans la formule (I) et peut également représenter un atome d'hydrogène,
   les composés de formule (VI), (VII) et (VIII) représentant le composé de formule (IX) :

   A-G₁-Cy-W₄H (IX)

   dans laquelle W₄ représente un atome d'oxygène ou de soufre, ou un groupement NH ou NRₐ (où Rₐ est défini comme précédemment),
   composé de formule (IX) sur lequel on condense :
   - un composé de formule (X) : 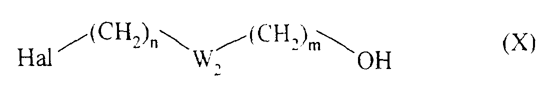 dans laquelle Hal représente un atome de brome, de chlore ou d'iode, et n, W₂ et m sont tels que définis dans la formule (I), (étant entendu que l'on ne peut pas avoir deux hétéroatomes consécutifs et que la chaîne ainsi définie peut comporter une ou plusieurs insaturations),
   - ou un composé de formule (XI) : 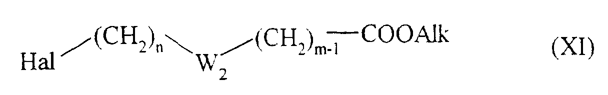 dans laquelle Hal, W₂, n et m sont définis comme précédemment et Alk représente un radical alkyle (étant entendu que l'on ne peut pas avoir deux hétéroatomes consécutifs et que la chaîne ainsi définie peut comporter une ou plusieurs insaturations), suivi d'une réduction,
      pour conduire au composé de formule (XII) :

      A-G₁ -Cy-W₄-(CH₂)ₙ-W₂-(CH₂)ₘ-OH (XII)

      dans laquelle A, G₁, Cy, W₂, W₄, n et m sont définis comme dans la formule (I) (étant entendu que l'on ne peut pas avoir deux hétéroatomes consécutifs dans l'enchaînement W₄-(CH₂)ₙ-W₂-(CH₂)ₘ-OH et que la chaîne ainsi définie peut comporter une ou plusieurs insaturations),
      dont la fonction hydroxyle est transformée classiquement en groupe partant comme par exemple un mésylate, un tosylate, ou un dérivé halogéné, pour conduire au composé de formule (XII') :

      A-G₁-Cy-W₄-(CH₂)ₙ-W₂-(CH₂)ₘ-E (XII')

      dans laquelle A, G₁, Cy, W₄, n, W₂ et m sont définis comme précédemment et E représente un groupement mésyle ou tosyle ou un atome d'halogène,
      sur lequel on fait agir un composé de formule (XIII) :

      B-G₃-Cy-W'₄H (XIII)

      dans laquelle B, G₃ et Cy sont définis comme dans la formule (I) et W'₄ peut prendre les mêmes valeurs que W₄ défini précédemment,
      pour conduire au composé de formule (I/a), cas particulier des composés de formule (I) :

      A-G₁-Cy-W₄-(CH₂)ₙ-W₂-(CH₂)ₘ-W'₄-Cy-G₃-B (I/a)

      dans laquelle A, G₁, Cy, W₄, n, W₂, m, W'₄, G₃ et B sont tels que définis précédemment,
      (les composés de formule (I/a) pour lesquels les groupements A-G₁-Cy-W₄- et W'₄-Cy-G₃-B sont identiques pouvant être obtenus directement à partir du composé de formule (IX) sur lequel on condense en milieu basique un composé de formule (X') : 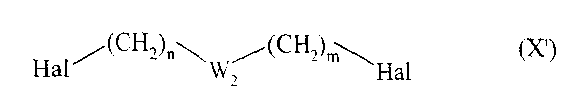 dans laquelle Hal, n, m et W₂ ont la même définition que précédemment),
◆ ou transformé en utilisant par exemple le phényl bis (trifluorométhane-sulfonimide) en milieu basique en trifluorométhane sulfonate correspondant de formule (XIV) :

   A-G₁-Cy-OSO₂CF₃ (XIV)

   dans laquelle A, G₁ et Cy sont définis comme précédemment,
   - sur lequel on fait agir, dans des conditions de catalyse par un dérivé du palladium approprié, un dérivé de l'acide borique (R_{b}B(OH)₂) ou un dérivé de l'étain (R_{b}SnBu₃) (où R_{b} représente un groupement de formule (XV) :

      B-G₃-Cy-W₃-(CH₂)ₘ-W₂-(CH₂)ₙ-CH₂- (XV)

      dans laquelle B, G₃, Cy, W₃, m, W₂ et n sont tels que définis précédemment, étant entendu que l'on ne peut pas avoir deux hétéroatomes consécutifs dans l'enchaînement -W₃-(CH₂)ₘ-W₂- et que la chaîne ainsi définie peut comporter une ou plusieurs insaturations),
      pour conduire au composé de formule (I/b), cas particulier des composés de formule (I) :

      A-G₁-Cy-CH₂-(CH₂)ₙ-W₂-(CH₂)ₘ-W₃-Cy-G₃-B (I/b)

      dans laquelle A, G₁, Cy, n, W₂, m, W₃, Cy, G₃ et B sont définis comme précédemment (étant entendu que l'on en peut pas avoir deux hétéroatomes consécutifs dans l'enchaînement -W₂-(CH₂)ₘ-W₃- et que la chaîne ainsi définie peut comporter une ou plusieurs insaturations),
      les composés de formule (I/c), cas particulier des composés de formule (I) :

      A-G₁-Cy-W₁-(CH₂)ₙ-W₂-(CH₂)ₘ-CH₂-Cy-G₃-B (I/c)

      dans laquelle A, G₁, Cy, W₁, n, W₂, m, G₃ et B sont définis comme précédemment (étant entendu que l'on ne peut pas avoir deux hétéroatomes consécutifs dans l'enchaînement -W₁-(CH₂)ₙ-W₂- et que la chaîne ainsi définie peut comporter une ou plusieurs insaturations),
      étant obtenus selon un mode opératoire similaire à partir du composé de formule (XIV') :

      B-G₃-Cy-OSO₂CF₃ (XIV')

      dans laquelle B, G₃ et Cy sont tels que définis précédemment,
   - ou que l'on place, dans des conditions de couplage en utilisant des dérivés du Nickel ou du palladium par exemple, en présence d'un composé de formule (XIV') afin de conduire au composé de formule (I/d), cas particulier des composés de formule (I) :

      A-G₁-Cy-Cy-G₃-B (I/d)

      dans laquelle A, G₁, Cy, G₃ et B sont tels que définis précédemment.
   l'ensemble des composés (I/a) à (I/d) formant le composé de formule (I) qui peut être purifié si on le désire par une technique classique de purification, dont on sépare éventuellement les isomères selon une technique classique de séparation et que l'on transforme, le cas échéant, en leurs sels d'addition à un acide ou à une base pharmaceutiquement acceptable.

Les composés de formule (V) sont aisément accessibles à l'homme du métier selon des méthodes décrites dans la littérature.

Les composés de l'invention et les compositions pharmaceutiques les contenant s'avèrent être utiles pour le traitement des troubles du système mélatoninergique.

L'invention concerne également les composés de formule (XII") :

A-G₁-Cy-W₄-(CH₂)ₙ-W₂-(CH₂)ₘ-E' (XIIʺ)

dans laquelle A, G₁, Cy, W₄, n, W₂ et m sont tels que définis précédemment et E' représente un groupement hydroxyle ou un atome d'halogène (fluor, chlore, brome ou iode),
étant entendu que
- lorsque Cy représente un naphtalène et G₁-A représente un groupement -(CH₂)₂-NR¹C(Q)R² ou -(CH₂)₂-NR¹C(Q)NR²R³ (ou R¹, R² et R³ sont tels que définis précédemment), alors la chaîne -W₄-(CH₂)ₙ-W₂-(CH₂)ₘ- ne peut représenter une chaîne -O-alkyle-,
- le composé de formule (XII") ne peut représenter le *N*-{2-[5-(2-hydroxvéthoxy)-*1H*-indol-3-yl]éthyl} acétamide.
leurs énantiomères et diastéréoisomères, ainsi que leurs sels d'addition à un acide ou une base pharmaceutiquement acceptable,
en tant qu'intermédiaires de synthèse mais également en tant que composés utiles pour le traitement des troubles liés au système mélatoninergique.

L'étude pharmacologique des dérivés de l'invention a en effet montré qu'ils étaient atoxiques. doués d'une haute affinité pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immmunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Les exemples suivants illustrent l'invention et ne la limitent en aucune façon. Les préparations suivantes conduisent à des composés de l'invention ou à des intermédiaires de synthèse utiles dans la préparation de l'invention.

### Préparation 1 : N-[2-(7-Hydroxy-1-naphtyl)éthyl]-acétamide

Sous atmosphère inerte, 27,5 mmol du complexe tribromure de bore/diméthylsulfure sont dissoutes dans 100 ml de dichlorométhane et agitées pendant 15 minutes à température ambiante. Une solution de 13,7 mmol de *N*-[2-(7-méthoxy-1-naphtyl)éthyl]acétamide dans 50 ml de dichlorométhane est ajoutée, et le milieu réactionnel est porté à reflux pendant 30 heures. Après refroidissement, la réaction est hydrolysée avec précaution et le dichlorométhane est évaporé. Le milieu est alors extrait à l'acétate d'éthyle, les phases organiques regroupées sont lavées par une solution aqueuse de bicarbonate de potassium 1M. La phase organique est séchée sur sulfate de magnésium, et concentrée pour conduire au composé du titre. Solide blanc.
Point de fusion : 125-126°C

En procédant comme dans la Préparation 1 à partir du substrat approprié, on obtient les Préparations 2 à 19

### Préparation 2 : N-Butyl-N'-[2-(7-hydroxy-1-naphtyl)éthyl]urée

### Préparation 3 : N-[2-(7-Hydroxy-1-naphtyl)éthyl]cyclopropane carboxamide

### Préparation 4 : 4-(7-Hydroxy-1-naphtyl)-N-méthyl butanamide

### Préparation 5 : N-[2-(7-Hydroxy-1-naphtyl)éthyl]-3-buténamide

### Préparation 6 : N-[2-(7-Hydroxy-3-phényl-1-naphtyl)éthyl]acétamide

### Préparation 7 : N-[2-(5-Hydroxy-1-benzothiophèn-3-yl)éthyl]butanamide

### Préparation 8 : 2,2,2-Trifluoro-N-[2-(5-hydroxy-1-henzothiophèn-3-yl)éthyl] acétamide

### Préparation 9 : 4-(5-Hydroxy-1-benzofuran-3-yl)-N-méthyl butanamide

### Préparation 10 : N-[2-(5-Hydroxy-1-benzofuran-3-yl)éthyl]cyclopropane carboxamide

### Préparation 11 : N-[2-(5-Hydroxy-1-benzofuran-3-yl)éthyl]-N'-propylurée

### Préparation 12 : N-{2-[5-Hydroxy-2-(3-méthoxybenzyl)-1-benzofuran-3-yl]éthyl}acétamide

### Préparation 13 : N-[2-(5-Hydroxy-1H-indol-3-yl)éthyl]-N'-propylthiourée

### Préparation 14 : N-[2-(5-Hydroxy-1H-indol-3-yl)éthyl]cyclobutane carboxamide

### Préparation 15 : N-[2-(5-Hydroxy-1-méthyl-1H-pyrrolo[2,3-b]pyridin-3-yl)éthyl] acétamide

### Préparation 16 : N-[2-(5-Hydroxy-1-méthyl-1H-pyrrolo[2,3-b]pyridin-3-yl)éthyl]-N'-propylurée

### Préparation 17 : N-[2-(6-Hydroxy-3,4-dihydro-2H-chromèn-4-yl)éthyl]acétamide

### Préparation 18 : N-[(6-Hydroxy-2H-chromèn-3-yl)méthyl]butanamide

### Préparation 19 : N-[(7-Hydroxy-1,4-benzodioxin-2-yl)méthyl]-N'-propylurée

### Préparation 20 : N-[2-(7-Mercapto-1-naphtyl)éthyl]benzamide

### Stade A : N-[2-(7-Hydroxy-1-naphyl)éthyl]benzamide

On procède comme dans la Préparation 1 à partir du *N*-[2-(7-Méthoxy-1-naphtyl)éthyl]benzamide.

### Stade B : N-[2-(7-Mercapto-1-naphtyl)éthyl]benzamide

A une solution d'hydroxyde de potassium (10 mmol) dissoute dans 15 ml d'eau et 16 ml de tétrahydrofurane, on additionne le produit obtenu au stade A (9 mmol) en maintenant l'agitation. La solution est refroidie à l'aide d'un bain de glace et de sel et on ajoute goutte à goutte le chlorure diméthylthiocarbamoyle (9 mmol) en solution dans le tétrahydrofurane (15 ml) sous agitation. Après une demi-heure d'agitation en maintenant le froid, le milieu réactionnel est extrait au chloroforme. Les phases organiques sont regroupées, séchées sur sulfate de magnésium, filtrées puis concentrées sous pression réduite. Le résidu est repris dans le diphényléther (10 ml) et porté au reflux pendant une heure sous atmosphère d'azote. Le diphényléther est évaporé sous pression réduite jusqu'à obtention d'une solution d'environ 2 ml. Les ml de distillat encore chauds sont versés avec précaution dans 50 ml d'hexane pour donner après refroidissement un solide isolé par filtration. Le solide ainsi collecté est additionné à une solution d'hydroxyde de potassium (380 mg) dissous dans un mélange eau/méthanol (1 ml/10 ml). La solution est portée au reflux pendant 12 heures puis refroidie et concentrée sous pression réduite. Le résidu est repris avec 20 ml de chloroforme et extrait 3 fois à l'eau. La phase organique est séchée sur sulfate de magnésium, filtrée et concentrée sous pression réduite. Le résidu est chromatographié sur gel de silice pour conduire au produit du titre.

### Préparation 21 : 2-Phényl-N-[2-(5-mercapto-1-benzofuran-3-yl)éthyl]acétamide

On procède comme dans la Préparation 20 à partir du 2-phényl-*N*-[2-(5-hydroxy-1-benzofuran-3-yl)éthyl]acétamide.

### Préparation 22 : N-[2-(5-Amino-1-benzothiophèn-3-yl)éthyl]cyclohexane carboxamide

### Stade A :N-[2-(5-Hydroxy-1-benzothiophèn-3-yl)éthyl]cyclohexane carboxamide

On procède comme dans la Préparation 1 à partir du *N*-[2-(5-méthoxy-1-benzothiophèn-3-yl)éthyl]cyclohexane carboxamide.

### Stade B : N-[2-(5-Bromo-1-benzothiophèn-3-yl)éthyl]cyclohexane carboxamide

Dans un tricol de 150 ml équipé d'une ampoule à brome, d'un réfrigérant surmonté d'un tube rempli de chlorure de calcium, et d'un agitateur mécanique, on verse de la triphénylphosphine (10 mmol) et de l'acétonitrile (70 ml). La solution est refroidie à l'aide d'un bain de glace en maintenant l'agitation et on additionne le brome (10 mmol). A la fin de l'addition, le bain de glace est retiré puis on ajoute le produit obtenu au stade A (8 mmol). Le mélange réactionnel est agité à 60-70°C jusqu'à disparition du produit de départ. En fin de réaction, le mélange est filtré puis le filtrat est concentré sous pression réduite. Le résidu est repris dans l'acétate d'éthyle, lavé à l'eau puis avec une solution saturée d'hydrogénocarbonate de potassium, et encore une fois à l'eau puis séché sur sulfate de magnésium et concentré sous pression réduite. Le résidu est filtré sur gel de silice pour conduire au produit du titre.

### Stade C : N-[2- (5-Iodo-1-benzothiophèn-3-yl)éthyl]cyclohexane carboxamide

Un mélange du produit obtenu au stade B (2 mmol), d'iodure de potassium (30 mmol) et d'iodure de cuivre I (10 mmol) dans l'hexaméthyl phosphoramide (6 ml) est chauffé à 150-160°C avec agitation sous atmosphère d'azote jusqu'à ce qu'un taux de conversion de 90 % soit atteint. On ajoute alors de l'acide chlorhydrique dilué puis de l'éther et la mixture est alors filtrée pour éliminer les sels de cuivre I insolubles. La phase organique est séparée, lavée avec une solution de sulfite de sodium, de l'eau, séchée sur sulfate de magnésium et évaporée pour donner un résidu que l'on chromatographie sur gel de silice pour conduire au produit du titre.

### Stade D : N-[2(5-Vinyl-1-benzothiophèn-3-yl)éthyl]cyclohexane carboxamide

15 mmol du produit obtenu au stade C, 16 mmol de vinyl tributylétain et 0,43 mmol de (triphénylphosphine) palladium tetrakis, sont portés sous agitation à 110°C pendant 3 heures dans 30 ml de *N*-méthylpyrrolidinone. Après évaporation du solvant, le résidu est repris dans 20 ml de dichlorométhane et traité par une solution aqueuse à 10 % de fluorure de potassium. Après extraction, concentration sous pression réduite et chromatographie sur gel de silice, on obtient le produit du titre pur.

### Stade E : N-[2-(5-Formyl-1-benzothiophèn-3-yl)éthyl]cyclohexane carboxamide

A une solution de 10 mmol du produit obtenu dans le stade D dans un mélange de 50 ml de dioxane et 25 ml d'eau sont ajoutés à température ambiante 1,10 g de tétroxyde d'osmium dans le 2-méthyl-2-propanol, puis 8,70 g de Periodate de sodium. Après agitation une nuit à température ambiante, la suspension est filtrée, le filtrat concentré sous pression réduite. Le résidu obtenu est repris dans le dichlorométhane. La phase organique est lavée avec de l'eau, séchée et évaporée. Le résidu est purifié par chromatographie sur gel de silice pour conduire au produit du titre.

### Stade F : Acide 3-{2-[(Cyclohexylcarbonyl)amino]éthyl}-1-benzothiophène-5-carboxylique

A une solution de 6,88 mmol du produit obtenu dans le stade E dans 30 ml d'acétone sont ajoutés à température ambiante 2,7 g de permanganate de potassium dans 50 ml d'un mélange acétone/eau (50/50). La solution est agitée 2 heures à température ambiante puis filtrée. Le filtrat est concentré sous pression réduite et chromatographié sur gel de silice pour conduire au produit du titre.

### Stade G : Chlorure de l'acide 3-{2-[(cyclohexylcarbonyl)amino]éthyl}-1-benzothiophène-5-carboxylique

5 mmol du produit obtenu dans le stade F sont dissoutes dans 40 ml de chlorure de thionyle. Après agitation sous atmosphère inerte pendant 1 heure, le chlorure de thionyle est évaporé sous pression réduite pour conduire au produit du titre.

### Stade H : N-[2-(5-Amino-1-benzothiophèn-3-yl)éthyl]cyclohexane carboxamide

Une solution du produit obtenu dans le stade G (20 mmol) dans le dichlorométhane (30 ml) contenant du bromure de tétrabutyl ammonium (20 mg) est refroidie dans un bain de glace. Après addition de l'azoture de sodium (25 mmol) dissous dans 5 ml d'eau la solution est agitée vigoureusement à 0°C pendant 2 heures. La phase organique est séparée, lavée à l'eau (2 x 5 ml) et séchée sur sulfate de magnésium. Après filtration. on ajoute l'acide trifluoroacétique (30 mmol) et la solution est agitée sous reflux pendant 60 heures. Après refroidissement, la phase organique est lavée avec une solution saturée d'hydrogénocarbonate de sodium (2 x 5 ml) et concentrée sous pression réduite. Le résidu est alors repris dans le méthanol (20 ml) et on ajoute de l'eau (80 ml) puis du carbonate de potassium (30 mmol). Après agitation à température ambiante pendant 20 heures, le milieu réactionnel est concentré sous pression réduite jusqu'à un volume de 60 ml environ puis extrait 3 fois à l'éther (3 x 50 ml). Après séchage sur sulfate de sodium, la phase organique est filtrée puis évaporée sous pression réduite. Le résidu est chromatographié sur gel de silice pour conduire au produit du titre.

### Préparation 23 : 2-(5-Amino-1-benzofuran-3-yl)-N-hexyl acétamide

On procède comme dans la Préparation 22.

### Préparation 24 : 8-[2-(Acétylamino)éthyl]-2-naphtyl trifluorométhanesulfonate

A une solution de 0,07 mol du composé obtenu dans la Préparation 1 dans un litre de dichlorométhane sont ajoutés 60 ml de triéthylamine. Le milieu réactionnel est porté à reflux jusqu'à solubilisation, puis 0,1 mol de phényl bis (trifluorométhanesulfonimide) et 0,75 mol de carbonate de potassium sont ajoutés. Après 4 heures à reflux, le milieu est lavé par un litre d'hydrogénocarbonate de sodium 1M puis par un litre d'acide chlorhydrique 1M. La phase organique est séchée, concentrée et purifiée par chromatographie sur gel de silice pour conduire au produit du titre.

### Préparation 25 : 3-{2-[(Cyclopropylcarbonyl)amino]éthyl}-1-benzofuran-5-yl trifluorométhanesulfonate

On procède comme dans la Préparation 24 à partir du produit obtenu dans la Préparation 10.

### Préparation 26 : 3-[2-(Acétylamino)éthyl]-1-méthyl-1H-pyrrolo[2,3-b]pyridin-5-yl-trifluorométhanesulfonate

On procède comme dans la Préparation 24 à partir du composé obtenu dans la Préparation 15.

### Préparation 27 : N-[2-(7-Hydroxy-1,2,3,4-tétrahydro-1-naphtalényl)éthyl]acétamide

On procède comme dans la Préparation 1 à partir du *N*-[2-(7-méthoxy-1,2,3A-tétrahydro-1-naphtalényl)éthyl]acétamide.
Point de fusion : 149-150°C

### Préparation 28 : N-{2-[5-Hydroxy-1-(phénylsulfonyl)-1H-indol-3-yl]éthyl}acétamide

### Stade A : N-{2-[5-Méthoxy-1-(phénylsulfonyl)-1H-indol-3-yl]éthyl}acétamide

Dissoudre 5 g (21,5 mmol) de mélatonine dans 150 ml de dichlorométhane. Sous agitation ajouter 3,41 g (84 mmol) de soude et 0,35 g (0,9 mmol) d'hydrogénosulfate de tétrabutylammonium. Refroidir le milieu dans un bain de glace, et ajouter goutte à goutte 4,06 ml (31,5 mmol) de chlorure de benzènesulfonyle. Après une nuit d'agitation à température ambiante, filtrer l'excès de soude et le catalyseur. Evaporer le solvant sous vide, et recristalliser le solide obtenu.
Point de fusion : 140-141°C

### Stade B : N-{2-[5-Hydroxy-1-(phénylsulfonyl)-1H-indol-3-yl]éthyl}acétamide

On procède comme dans la Préparation 1 à partir du composé obtenu au stade A.
Point de fusion : 205-206°C

### Préparation 29 : N-[2-(5-Hydroxy-1-benzofuran-3-yl)éthyl]acétamide

On procède comme dans la Préparation 1 à partir du *N*-[2-(5-méthoxy-1-benzofuran-3-yl)éthyl]acétamide.
Point de fusion : 140°C

### Préparation 30 : N-[2-(5-Hydroxy-1H-indol-3-yl)éthyl]acétamide

On procède comme dans la Préparation 1 à partir de la mélatonine.
Huile incolore.

### Préparation 31 : 2-(7-Hydroxy-1-naphtyl)éthyl carbamate de tert-butyle

Dans un ballon de 100 ml, mettre en suspension le bromhydrate du 8-(2-aminoéthyl)-2-naphtol (3 g, 1,12 mmol) dans 30 ml de dichlorométhane et ajouter la triéthylamine (3,88 ml, 2,8 mmol). Refroidir le milieu réactionnel à 0°C à l'aide d'un bain de glace, ajouter goutte à goutte le dicarbonate de di-*tert*-butyle (2,2 g, 1 mmol) dissout dans 10 ml de dichlorométhane. Laisser agiter le milieu réactionnel à température ambiante pendant 4 heures. Laver le milieu réactionnel avec une solution aqueuse d'acide chlorhydrique 0,5M puis à l'eau. Sécher la phase organique sur du sulfate de magnésium et l'évaporer sous pression réduite. Recristalliser le résidu obtenu dans du cyclohexane-toluène (1/10).
Point de fusion : 72-73°C

### Préparation 32 : N-[2-(5-Hydrox-1-méthyl-1H-pyrrolo[3,2-b]pyridin-3-yl)éthyl] acétamide

On procède comme dans la Préparation 1 à partir du *N*-[2-(5-méthoxy-1-méthyl-*1H*-pyrrolo*[3,2-b]*pyridin-3-yl)éthyl]acétamide.

### Préparation 33 : 3-[2-(Acétylamino)éthyl]-1H-indol-5-yl trifluorométhanesulfonate

On procède comme dans la Préparation 24 à partir du composé obtenu dans la Préparation 30.

### Préparation 34 : N-[2-(5-Hydroxy-1-benzothiophèn-3-yl)éthyl]acétamide

On procède comme dans la Préparation 1 à partir du *N*-[2-(5-méthoxy-1-benzothiophèn-3-yl)éthyl]acétamide.
Point de fusion : 166-168°C

### Préparation 35 : : N-[2-(7-Hydroxy-2-méthoxy-1-naphtyl)éthyl]acétamide

On procède comme dans la Préparation 1 à partir du *N*-[2-(2,7-diméthoxy-1-naphtyl)éthyl]acétamide.

### Exemple 1 : N-(2-{7-[2-({8-[2-(Acétylamino)éthyl]-2-naphtyl}oxy)éthoxy]-1-naphtyl} éthyl)acétamide

### Stade A : N-{2-[7-(2-Bromoéthoxy)napht-1-yl]éthyl}acétamide

On dissout le composé obtenu dans la Préparation 1 (0,009 mol) dans 20 ml d'un mélange de diméthylsulfoxyde (6 ml) et de butanone (14 ml). On ajoute 0,027 mol de carbonate de potassium et 0,036 mol de dibromoéthane, puis on chauffe à reflux pendant 48 heures. Le milieu réactionnel est ensuite refroidi et versé dans l'eau. La phase aqueuse est extraite par Et₂O, puis la phase organique est lavée à l'eau jusqu'à neutralité des eaux de lavage, puis séchée sur sulfate de magnésium et évaporée sous pression réduite. Le résidu obtenu est purifié par chromatographie sur gel de silice (éluant: acétone/cyclohexane (2/8)) et recristallisé. Solide blanc.
Point de fusion : 110-111°C

| Microanalyse élémentaire : | | | | |
|---|---|---|---|---|
| | % | C | H | N |
| Calculé | | 57,15 | 5,40 | 4,17 |
| Trouvé | | 57,28 | 5,38 | 3,91 |

### Stade B : N-(2-{7-[2-({8-[2-(Acétylamino)éthyl]-2-naphtyl}oxy)éthoxy]-1-naphtyl} éthyl)acétamide

Dans un ballon de 100 ml, on dissout 0,003 mol du composé obtenu dans la Préparation 1 et 0,003 mol du composé obtenu au stade A dans un mélange constitué de 3 ml de diméthylsulfoxyde et 20 ml de butanone. On ajoute 0.009 mol de carbonate de potassium et un cristal d'iodure de potassium puis on chauffe à reflux pendant 12 heures. Le milieu réactionnel est ensuite refroidi et versé dans 100 ml d'eau. Le précipité formé est essoré et recristallisé. Solide beige.
Point de fusion : 220-222°C

| Microanalyse élémentaire | | | | |
|---|---|---|---|---|
| | % | C | H | N |
| Calculé | | 71,69 | 6,61 | 5,57 |
| Trouvé | | 72,03 | 6,60 | 5,53 |

### Exemple 2 : N-{2-[7-(2-{[8-(2-{[(Butylamino)carbonyl]amino}éthyl)-2-naphtyl] oxy}éthoxy)-1-naphtyl]éthyl}acétamide

On procède comme dans l'Exemple 1 en remplaçant dans le stade B le naphtol obtenu dans la Préparation 1 par le composé obtenu dans la Préparation 2.

### Exemple 3 : N-(2-{7-[2-({8-[2-(3-Buténoylamino)éthyl]-2-naphtyl}oxy)éthoxy]-1-naphtyl}éthyl)-3-buténamide

On procède comme dans l'Exemple 1 en remplaçant le composé obtenu dans la Préparation 1 par le composé obtenu dans la Préparation 5.

### Exemple 4 : N-[2-(7-{[2-({8-[2-(Acétylamino)éthyl]-2-naphtyl}oxy)éthyl]thio}-1-naphtyl)éthyl]benzamide

On procède comme dans l'Exemple 1 en remplaçant dans le stade B le naphtol obtenu dans la Préparation 1 par le composé obtenu dans la Préparation 20.

### Exemple 5 : : N-[2-(5-{[2-({3-[2-(Butylamino)éthyl]-1-benzothiophèn-5-yl}oxy)éthyl] amino}-1-benzothiophèn-3-yl)éthyl]cyclohexane carboxamide

On procède comme dans l'Exemple 1 en remplaçant :
- dans le stade A, le composé obtenu dans la Préparation 1 par le composé obtenu dans la Préparation 7.
- dans le stade B, le composé obtenu dans la Préparation 1 par le composé obtenu dans la Préparation 22.

### Exemple 6 : N-(2-{7-[3-({8-[2-(Acétylamino)éthyl]-2-naphtyl}oxy)propoxy]-1-naphtyl}éthyl)acétamide

### Stade A : N-{2-[7-(3-Hydroxypropyloxy)napht-1-yl]éthyl}acétamide

Dans un ballon de 100 ml, on dissout 0,022 mol du composé obtenu dans la Préparation 1 dans 30 ml de diméthylformamide. On ajoute 0,066 mol de carbonate de potassium et 0,033 mol de 3-bromopropan-1-ol, puis le mélange est chauffé à 80°C pendant 4 heures. Le milieu réactionnel est refroidi et versé dans 100 ml d'une solution d'HCl 1M. La phase aqueuse est extraite 3 fois avec Et₂O puis la phase organique est séchée sur MgSO₄ et évaporée sous pression réduite. Le produit du titre est obtenu après recristallisation. Solide blanc. Point de fusion : 141-142°C

### Stade B : 3-({8-[2-(Acétylamino)éthyl]-2-naphtyl}oxy)propylméthanesulfonate

Dans un ballon de 250 ml, l'alcool obtenu au stade A est dissous dans 50 ml de dichlorométhane et on ajoute 0,012 mol de triéthylamine. On refroidit dans un bain de glace-sel à -10°C puis 0,012 mol de chlorure de mésyle sont ajoutées goutte à goutte sous agitation magnétique. Le milieu réactionnel est agité à température ambiante pendant 4 heures. Puis on additionne 100 ml d'eau et on poursuit par une extraction au CH₂Cl₂. La phase organique est lavée à l'eau, séchée sur MgSO₄ et évaporée sous pression réduite. L'huile obtenue est purifiée par chromatographie sur gel de silice (éluant : acétone/cyclohexane (2/8)).

### Stade C : N-(2-{7-[3-({8-[2-(Acétylamino)éthyl]-2-naphtyl}oxy)propoxy]-1-naphtyl}éthyl)acétamide

Dans un ballon de 100 ml contenant 30 ml de méthanol, on ajoute par petites portions 0,06 g de sodium. Lorsque le sodium est totalement consommé, on ajoute 0,0033 mol du composé obtenu dans la Préparation 1 et on laisse agiter pendant 20 minutes. Le méthanol est évaporé sous pression réduite et le résidu est repris dans 15 ml de DMF, puis on ajoute 0,0027 mol du composé obtenu au stade B. Le milieu réactionnel est ensuite chauffé à reflux pendant 12 heures puis refroidi et versé dans 100 ml d'eau et 10 ml d'HCl 3M. Après extraction à l'acétate d'éthyle, la phase organique est lavée avec une solution de soude 10 % puis à l'eau. Après séchage sur MgSO₄, évaporation sous pression réduite du solvant, le composé du titre est obtenu par recristallisation. Solide beige.
Point de fusion : 101-103°C

| Microanalyse élémentaire : | | | | |
|---|---|---|---|---|
| | % | C | H | N |
| Calculé | | 74,67 | 6,87 | 5,18 |
| Trouvé | | 74.31 | 6,87 | 5,15 |

### Exemple 7 : N-(2-{7-[3-({8-[2-(Acétylamino)éthyl]-2-naphtyl}oxy)propoxy]-3-phényl-1-naphtyl}éthyl)acétamide

On procède comme dans l'Exemple 6 en remplaçant dans le stade C le composé obtenu dans la Préparation 1 par le composé obtenu dans la Préparation 6.

### Exemple 8 : N-Méthyl-4-{7-[3-({8-[4-(méthylamino)-4-oxopropyl]-2-naphtyl}oxy) propoxy]-1-naphtyl}butanamide

On procède comme dans l'Exemple 6 en remplaçant le produit de la Préparation 1 par le composé obtenu dans la Préparation 4.

### Exemple 9 : N-(2-{5-[3-({3-[4-(Méthylamino)-4-oxobutyl]-1-benzofuran-5-yl}oxy) propoxy-1-benzofuran-3-yl}éthyl)cyclopropane carboxamide

On procède comme dans l'Exemple 6 en remplaçant :
- dans le stade A, le composé de la Préparation 1 par le composé de la Préparation 9,
- dans le stade C, le composé de la Préparation 1 par le composé de la Préparation 10.

### Exemple 10 : N-{2-[1-Méthyl-5-(3-{[1-méthyl-3-(2-{[(propylamino)carbonyl]amino} éthyl)-1H-pyrrolo[2,3-b]pyridin-5-yl]oxy}propoxy)-1H-pyrrolo[2,3-b] pyridin-3-yl]étliyl}acétamide

On procède comme dans l'Exemple 6 en remplaçant :
- dans le stade A, le composé de la Préparation 1 par le composé de la Préparation 15,
- dans le stade C, le composé de la Préparation 1 par le composé de la Préparation 16.

### Exemple 11 : N-(2-{6-[3-({4-[2-(Acétylamino)éthyl]-3,4-dihydro-2H-chromèn-6-yl}oxy)propoxy]-3,4-dihydro-2H-chromèn-4-yl}éthyl)acétamide

On procède comme dans l'Exemple 6 en remplaçant le composé de la Préparation 1 par le composé de la Préparation 17.

### Exemple 12 : N-(2-{7-[4-({8-[2-(Acétylamino)éthyl]-2-naphtyl}oxy)butoxy]-1-naphtyl}éthyl)acétamide

### Stade A : 4-({8-[2-(Acétylamino)éthyl]-2-naphtyl}oxy)butanoate d'éthyle

Dans un ballon de 100 ml, on dissout 0,022 mol du composé obtenu dans la Préparation 1 dans 50 ml d'acétonitrile. On ajoute 0,066 mol de carbonate de potassium et la réaction est agitée à 80°C pendant 30 minutes. 0,033 mol de 1-bromobutyrate d'éthyle sont ensuite ajoutées goutte à goutte et la réaction est agitée 1 heure à 80°C. L'acétonitrile est évaporé sous pression réduite et le résidu solubilisé dans une solution d'HCl 1N. Après extraction à l'acétate d'éthyle, lavage de la phase organique à l'eau. séchage sur MgSO₄ et évaporation sous pression réduite, le composé du titre est purifié par recristallisation. Solide beige.
Point de fusion : 64-66°C

### Stade B : N-{2-[7-(4-Hydroxybutyloxy)napht-1-yl]éthyl}acétamide

Dans un ballon de 250 ml, l'ester obtenu au stade A (0,009 mol) est dissous dans 100 ml d'éther anhydre. 0,009 mol d'hydrure mixte de lithium et d'aluminium sont ajoutées par portions et la réaction est agitée 6 heures à l'ambiante. Le milieu réactionnel est ensuite hydrolysé par quelques gouttes de NaOH 1M et le précipité formé est filtré. Le filtrat est séché sur MgSO₄ et évaporé sous pression réduite. Le résidu obtenu est précipité dans un mélange Et₂O/Ether de pétrole (1/1), essoré et recristallisé. Solide blanc.
Point de fusion : 82-84°C

| Microanalyse élémentaire : | | | | |
|---|---|---|---|---|
| | % | C | H | N |
| Calculé | | 71,73 | 7,69 | 4,64 |
| Trouvé | | 72,00 | 7,58 | 4,45 |

### Stade C : 4-({8-[2-(Acétylamino)éthyl]-2-naphtyl}oxy)butyl méthanesulfonate

On procède comme dans le stade B de l'Exemple 6 à partir du composé obtenu au stade B.

### Stade D : N-(2-{7-[4-({8-[2-(Acétylamino)éthyl]-2-naphtyl}oxy)butoxy]-1-naphtyl}éthyl)acétamide

On procède comme dans le stade C de l'Exemple 6. Solide beige.
Point de fusion : 176-178°C

| Microanalyse élémentaire : | | | | |
|---|---|---|---|---|
| | % | C | H | N |
| Calculé | | 74,97 | 7,08 | 5,46 |
| Trouvé | | 75,17 | 7,01 | 5,21 |

### Exemple 13 : N-(2-{7-[4-({8-[4-(Méthylamino)-4-oxobutyl]-2-naphtyl}oxy)butoxy]-1-naphtyl}éthyl)-3-buténamide

On procède comme dans l'Exemple 12 en remplaçant dans le stade A le composé de la Préparation 1 par le composé de la Préparation 4, et dans le stade D le composé de la Préparation 1 par le composé de la Préparation 5.

### Exemple 14 : N-(2-{7-[4-({8-[2-(Acétylamino)éthyl]-2-naphtyl}oxy)butoxy]-1-naphtyl}éthyl)cyclopropane carboxamide

On procède comme dans l'Exemple 12 en remplaçant dans le stade D le composé de la Préparation 1 par le composé de la Préparation 3.

### Exemple 15 : 2,2,2-Trifluoro-N-[2-(5-{4-[(3-{2-[(2,2,2-trifluoroacétyl)amino]éthyl}-1-benzothiophèn-5-yl)oxy]butoxy}-1-benzothiophèn-3-yl)éthyl] acétamide

On procède comme dans l'Exemple 12 en remplaçant le composé de la Préparation 1 par le composé de la Préparation 8.

### Exemple 16 : N-({6-[4-({3-[(Butyrylamino)méthyl]-2H-chromèn-6-yl}oxy)butoxy]-2H-chromèn-3-yl}méthyl)butanamide

On procède comme dans l'Exemple 12 en remplaçant le composé de la Préparation 1 par le composé de la Préparation 18.

### Exemple 17 : N-[2-(7-{[6-({8-[2-(Acétylamino)éthyl]-2-naphtyl}oxy)hexyl]oxy)-1-naphtyl)éthyl] acétamide

### Stade A : N-(2-{7-[(6-Hydroxyhexyl)oxy]-1-naphtyl}éthyl)acétamide

On procède comme dans le stade A de l'Exemple 6 en remplaçant le 3-bromopropan-1-ol par le 6-bromohexan-1-ol. Solide blanc.
Point de fusion : 58-61°C

| Microanalyse élémentaire : | | | | |
|---|---|---|---|---|
| | % | C | H | N |
| Calculé | | 72,91 | 8,41 | 4,25 |
| Trouvé | | 73,22 | 8,17 | 4,02 |

### Stade B : 6-({8-[2-(Acétylamino)éthyl]-2-naphtyl}oxy)hexyl méthanesulfonate

On procède comme dans le stade B de l'Exemple 6. Solide blanc.
Point de fusion : 66-67°C

### Stade C : N-[2-(7-{[6-({8-[2-(Acétylamino)éthyl]-2-naphtyl}oxy)hexyl]oxy}-1-naphtyl)éthyl]acétamide

On procède comme dans le stade C de l'Exemple 6. Solide blanc.
Point de fusion : 142-144°C

| Microanalyse élémentaire : | | | | |
|---|---|---|---|---|
| | % | C | H | N |
| Calculé | | 75,52 | 7,46 | 5,18 |
| Trouvé | | 75,32 | 7,59 | 4,96 |

### Exemple 18 : N-[2-(7-{[6-({8-[2-(Acétylamino)éthyl]-6-phényl-2-naphtyl}oxy)hexyl] oxy}-3-phényl-1-naphtyl)éthyl]acétamide

On procède comme dans l'Exemple 17 en remplaçant le composé obtenu dans la Préparation 1 par le composé obtenu dans la Préparation 6.

### Exemple 19 : 2-Phényl-N-{2-[5-({6-[(3-{2-[(2-phénylacétyl)amino]éthyl}-1-benzofuran-5-yl)thio]hexyl}thio)-1-benzofuran-3-yl]éthyl}acétamide

On procède comme dans l'Exemple 17 en remplaçant le composé de la Préparation 1 par le composé de la Préparation 21.

### Exemple 20 : N-Hexyl-2-{5-[(6-{[3-(2-{[(propylamino)carbonyl]amino}éthyl)-1-benzofuran-5-yl]oxy}hexyl)amino]-1-benzofuran-3-yl}acétamide

On procède comme dans l'Exemple 17 en remplaçant :
- dans le stade A, le composé de la Préparation 1 par le composé de la Préparation 23,
- dans le stade C, le composé de la Préparation 1 par le composé de la Préparation 11.

### Exemple 21: N-{2-[5-[(6-{[3-[2-(Acétylamino)éthyl]-2-(3-méthoxybenzyl)-1-benzofuran-5-yl]oxy}hexyl)oxy]-2-(3-méthoxybenzyl)-1-benzofuran-3-yl]éthyl}acétamide

On procède comme dans l'Exemple 17 en remplaçant le composé de la Préparation 1 par le composé de la Préparation 12.

### Exemple 22 : N-{2-[5-({6-[(3-{2-[(Cyclobutylcarbonyl)amino]éthyl}-1H-indol-5-yl)oxy]hexyl}oxy)-1H-indol-3-yl]éthyl}cyclobutane carboxamide

On procède comme dans l'Exemple 17 en remplaçant le composé de la Préparation 1 par le composé de la Préparation 14.

### Exemple 23 : N-(2-{5-[(6-{[3-(2-{[(Propylamino)carbothioyl]amino}éthyl)-1H-indol-5-yl]oxy}hexyl)oxy]-1H-indol-3-yl}éthyl)cyclobutane carboxamide

On procède comme dans l'Exemple 17 en remplaçant :
- dans le stade A, le composé de la Préparation 1 par le composé de la Préparation 14,
- dans le stade C, le composé de la Préparation 1 par le composé de la Préparation 13.

### Exemple 24 : N'-Propyl-N-({7-[(6-{[3-({[(propylamino)carbonyl]amino}méthyl)-1,4-benzodioxin-6-yl]oxy}hexyl)oxy]-1,4-benzodioxin-2-yl}méthyl)urée

On procède comme dans l'Exemple 17 en remplaçant le composé de la Préparation 1 par le composé de la Préparation 19.

### Exemple 25 : N-[2-(7-{8-[2-(Acétylamino)éthyl]-2-naphtyl}-1-naphtyl)éthyl] acétamide

Sous azote, 5,53 mmoles du composé obtenu dans la Préparation 24, 1,94 mmoles de dichlorobis (triphénylphosphine) nickel, 3,87 mmoles de triphénylphosphine et 8,30 mmoles de zinc sont mis en suspension dans 20 ml de DMF sec. Après chauffage 48 heures à 120°C sous azote, le milieu réactionnel est concentré avant de partager le résidu obtenu entre CH₂Cl₂ et NaHCO₃ 1M. La phase organique est ensuite séchée sur Na₂SO₄ et concentrée sous vide. Le composé du titre est obtenu après chromatographie sur gel de silice.
Point de fusion : 192,2-193,4°C

### Exemple 26 : N-(2-{5-(3-{2-[(Cyclopropylcarbonyl)amino]éthyl}-1-benzofuran-5-yl)-1-benzofuran-3-yl}éthyl)cyclopropane carboxamide

On procède comme dans l'Exemple 25 en remplaçant le composé obtenu dans la Préparation 24 par le composé obtenu dans la Préparation 25.

### Exemple 27 : N-{2-[5-{3-[2-(Acétylamino)éthyl]-1-méthyl-1H-pyrrolo[2,3-b]pyridin-5-yl}1-méthyl-1H-pyrrolo[2,3-b]pyridin-3-yl]éthyl}acétamide

On procède comme dans l'Exemple 25 en remplaçant le composé obtenu dans la Préparation 24 par le composé obtenu dans la Préparation 26.

### Exemple 28 : N [2-(7-{[5-({8-[2-(Acétylamino)éthyl]-2-naphtyl}oxy)pentyl]oxy}-1-naphtyl)éthyl]acétamide

### Stade A : 5-({8-[2-(Acétylamino)éthyl]-2-naphtyl}oxy)pentanoate de méthyte

Dans un ballon de 250 ml, dissoudre le composé obtenu dans la Préparation 1 (4.6 g ; 20 mmol) dans 70 ml d'acétonitrile. Ajouter le carbonate de potassium (8,3 g; 60 mmol) et laisser sous agitation magnétique à reflux pendant 30 minutes. Ajouter ensuite goutte à goutte le 5-bromovalérate de méthyle (3,4 ml, 24 mmol) et chauffer à reflux pendant 12 heures. Evaporer sous pression réduite l'acétonitrile. Reprendre le résidu par l'eau et extraire 3 fois par l'acétate d'éthyle. Laver la phase organique par une solution aqueuse d'acide chlorhydrique 1M, puis à l'eau jusqu'à neutralité des eaux de lavage, la sécher sur sulfate de magnésium et l'évaporer sous pression réduite. Faire précipiter l'huile obtenue dans un mélange éther éthylique-éther de pétrole (1-2). Essorer le précipité formé et le recristalliser dans un mélange toluène-cyclohexane (1/2).
Solide blanc.

### Stade B : N-(2-{7-[(5-Hydroxypentyl)oxy]-1-naphtyl}-éthyl)acétamide

Dans un ballon de 100 ml, dissoudre le composé obtenu au stade A (3,42 g, 10 mmol) dans 50 ml de tétrahydrofurane anhydre. Ajouter par petites portions l'hydrure mixte de lithium et d'aluminium (379,5 mg ; 10 mmol). Laisser agiter à température ambiante pendant 6 heures. Hydrolyser le milieu réactionnel par 100 ml d'une solution aqueuse d'acide chlorhydrique 1M. Extraire la phase aqueuse 3 fois par du dichlorométhane. Sécher la phase organique sur du sulfate de magnésium et l'évaporer sous pression réduite. Utiliser l'huile obtenue directement dans l'étape suivante.

### Stade C : 5-({8-[2-(Acétylamino)éthyl]-2-naphtyl}oxy)pentyl méthane sulfonate

Dans un ballon de 250 ml, dissoudre le composé obtenu au stade B (3,15 g, 10 mmol) dans 50 ml de dichlorométhane et ajouter la triéthylamine (1,6 ml ; 12 mmol). Refroidir dans un bain de glace-sel à 0°C, puis ajouter goutte à goutte et sous agitation magnétique le chlorure de mésyle (0,93 ml ; 12 mmol). Laisser revenir le milieu réactionnel à température ambiante et agiter pendant 5 heures. Ajouter 100 ml d'eau et extraire la phase aqueuse 3 fois par du dichlorométhane. Laver la phase organique par 3 fois 20 ml d'une solution aqueuse d'acide chlorhydrique 1M, puis à l'eau, la sécher sur du sulfate de magnésium et l'évaporer sous pression réduite. Purifier l'huile obtenue par chromatographie sur gel de silice (éluant : acétone/cyclohexane (3/7)).
Huile incolore.

### Stade D : N-[2-(7-{[5-({8-[2-(Acétylamino)éthyl]-2-naphtyl}oxy)pentyl]oxy}-1-naphtyl)éthyl]acétamide

Dans un ballon de 100 ml contenant 30 ml de méthanol, ajouter par petites portions le sodium (0,07 g; 0,0030 at. g.). Lorsque le sodium est totalement consommé, ajouter le naphtol (0,82 g, 3,6 mmol). Laisser sous agitation magnétique pendant 20 minutes. Evaporer sous pression réduite le méthanol. Reprendre le résidu obtenu par 15 ml de diméthylformamide. Ajouter le dérivé obtenu au stade C (1,2 g, 3 mmol) et chauffer à reflux pendant 12 heures. Laisser refroidir le milieu réactionnel et le verser dans un mélange de 100 ml d'eau et de 10 ml d'acide chlorhydrique 3M. Extraire 2 fois la phase aqueuse par de l'acétate d'éthyle. Laver la phase organique avec une solution aqueuse de soude à 10 % puis à l'eau. Sécher sur du sulfate de magnésium et évaporer sous pression réduite l'acétate d'éthyle. Recristalliser le résidu solide obtenu dans de l'acétonitrile. Point de fusion : 134-136°C

### Exemple 29 : N-[2-(7-{2-[2-({8-[2-(Acétylamino)éthyl]-2-naphtyl}oxy)éthoxy] éthoxy}-1-naphtyl)éthyl]acétamide

Dans un ballon de 100 ml, dissoudre le composé obtenu dans la Préparation 1 (1,14 g, 5 mmol) dans 50 ml d'acétonitrile. Ajouter le carbonate de potassium (0,83 g, 6 mmol) et laisser sous agitation magnétique à reflux pendant 30 minutes. Ajouter ensuite goutte à goutte le bis-(2-bromoéthyl)éther (0,25 ml, mmol) et chauffer à reflux pendant 12 heures. Evaporer sous pression réduite l'acétonitrile. Reprendre le résidu obtenu par une solution aqueuse de soude 1M. Filtrer le précipité obtenu, le laver à l'eau et le recristalliser dans l'acétonitrile puis l'acétate d'éthyle.
Point de fusion : 135-138°C

### Exemple 30 : N-(2-{7-{4-({8-[2-(Acétylamino)éthyl]-5,6,7,8-tétrahydro-2-naphtalényl}oxy)butoxy]-1,2,3,4-tétrahydro-1-naphtalényl}éthyl) acétamide

Dans un ballon de 100 ml, dissoudre le composé obtenu dans la Préparation 27 (0,8 g, 3,4 mmol) dans 50 ml d'acétonitrile. Ajouter le carbonate de potassium (0,57 g, 4,1 mmol) et laisser sous agitation magnétique à reflux pendant 30 minutes. Ajouter ensuite goutte à goutte le 1,4-dibromobutane (0,16 ml, 1,4 mmol) et chauffer à reflux pendant 12 heures. Evaporer sous pression réduite l'acétonitrile. Reprendre le résidu obtenu par une solution aqueuse de soude 1M. Filtrer le précipité obtenu, le laver à l'eau et le recristalliser dans l'acétonitrile.
Point de fusion : 119-121°C

### Exemple 31 : 2-{7-[4-({8-[2-(Acétylamino)éthyl]-2-naphtyl}oxy)butoxy]-1-naphtyl}éthyl carbamate de tert-butyle

Dans un ballon de 100 ml contenant 30 ml de méthanol, ajouter par petites portions le sodium (0,07 g ; 0,0030 at.g). Lorsque le sodium est totalement consommé, ajouter le composé obtenu dans la Préparation 31 (1 g, 3,6 mmol). Laisser sous agitation magnétique pendant 20 minutes. Evaporer sous pression réduite le méthanol. Reprendre le résidu obtenu par 15 ml de diméthylformamide. Ajouter le dérivé obtenu au stade C de l'Exemple 12 (1,1 g, 3 mmol) et chauffer à reflux pendant 12 heures. Laisser refroidir le milieu réactionnel et le verser dans un mélange de 100 ml d'eau et de 10 ml d'acide chlorhydrique 3M. Extraire 2 fois la phase aqueuse par de l'acétate d'éthyle. Laver la phase organique avec une solution aqueuse de soude à 10 % puis à l'eau. Sécher sur du sulfate de magnésium et évaporer sous pression réduite l'acétate d'éthyle. Recristalliser le résidu solide obtenu dans du toluène.
Point de fusion : 101-103°C

### Exemple 32 : N-{2-[5-(4-{[3-[2-(Acétylamino)éthyl]-1-(phénylsulfonyl)-1H-indol-5-yl]oxy}butoxy)-1-(phénylsulfonyl)-1H-indol-3-yl]éthyl}acétamide

Dissoudre 1 g (2,79 mmol) du composé obtenu dans la Préparation 28 dans 20 ml d'acétonitrile. Sous agitation, ajouter 0,38 g (2,79 mmol) de carbonate de potassium, et 0,13 ml (1,11 mmol) de 1,4-dibromobutane. Après une nuit à reflux, verser le milieu réactionnel dans 200 ml d'eau et de glace. Filtrer le précipité, laver à l'éther, sécher et recristalliser dans un mélange Dioxane/eau.
Point de fusion : 203-204°C

### Exemple 33 : N-(2-{5-[4-({3-[2-(Acétylamino)éthyl]-1-benzofuran-5-yl}oxy)butoxy]-1-benzofuran-3-yl}éthyl)acétamide

Dissoudre 0,70 g (3,19 mmol) du composé obtenu dans la Préparation 29 dans 20 ml d'acétonitrile. Sous agitation, ajouter 0,44 g (3,19 mmol) de carbonate de potassium et 0,15 ml (1,28 mmol) de 1,4-dibromobutane. Après une nuit à reflux, verser le milieu réactionnel dans 200 ml d'eau et de glace. Filtrer le précipité, laver à l'éther, sécher et recristalliser dans du toluène.
Point de fusion : 171-172°C

### Exemple 34 : N-(2-{5-[4-({3-[2-(Acétylamino)éthyl]-1H-indol-5-yl}oxy)butoxy]-1H-indol-3-yl}éthyl)acétamide

Dissoudre 1 g (4,58 mmol) du composé obtenu dans la Préparation 30 dans 20 ml d'acétonitrile. Sous agitation, ajouter 0,63 g (4,58 mmol) de carbonate de potassium, et 0,22 ml (1,83 mmol) de 1,4-dibromobutane. Après une nuit à reflux, verser le milieu réactionnel dans 200 ml d'eau et de glace. Filtrer le précipité, laver à l'acétone et sécher.
Point de fusion : 208-209°C

### Exemple 35 : N-{2-[7-(4-{[8-(2-Aminoéthyl)-2-naphtyl]oxy}butoxy)-1-naphtyl]éthyl} acétamide, chlorhydrate

Dans une fiole de 100 ml, mettre en suspension le composé obtenu dans l'Exemple 31 dans le méthanol. Faire barboter de l'acide chlorhydrique gaz jusqu'à l'obtention d'une phase limpide. Laisser agiter à température ambiante pendant 5 heures. Essorer le précipité formé et le recristalliser dans un mélange acétonitrile-méthanol (3/1).
Point de fusion : 188-189°C

### Exemple 36 : N-(2-{7-[4-({8-[2-(2-Furoylamino)éthyl]-2-naphtyl}oxy)butoxy]-1-naphtyl}éthyl)-2-furamide

### Stade A : 2-(7-{4-[(8-(2-[(Tert-butoxycarbonyl)amino]éthyl}-2-naphtyl)oxy] butoxy}-1-naphtyl)éthylcarbamate de tert-butyle

Dans un ballon de 100 ml, dissoudre le composé obtenu dans la Préparation 31 (5 mmol) dans 50 ml d'acétonitrile. Ajouter le carbonate de potassium (0,83 g, 6 mmol) et laisser sous agitation magnétique à reflux pendant 30 minutes. Ajouter ensuite goutte à goutte le 1,4-dibromobutane (0,37 ml, 2 mmol) et chauffer à reflux pendant 12 heures. Evaporer sous pression réduite l'acétonitrile. Reprendre le résidu obtenu par l'eau et extraire 3 fois par l'acétate d'éthyle. Laver la phase organique par une solution aqueuse de soude 1M, par une solution aqueuse d'acide chlorhydrique 1M, puis à l'eau jusqu'à neutralité des eaux de lavage, la sécher sur du sulfate de magnésium et l'évaporer sous pression réduite. Recristalliser le résidu obtenu dans du méthanol.
Point de fusion : 139-140°C

### Stade B : 2-[7-(4-{[8-(2-Aminoéthyl)-2-naphtyl]oxy}butoxy)-1-naphtyl]éthylamine, dichlorhydrate

Dans une fiole de 100 ml, mettre en suspension le composé obtenu au stade A (2 g, 3,2 mmol) dans le méthanol. Faire barboter de l'acide chlorhydrique gaz jusqu'à l'obtention d'une phase limpide. Laisser agiter à température ambiante pendant 5 heures. Essorer le précipité formé et le recristalliser dans l'acétonitrile.
Point de fusion : > 240°C

### Stade C : N-(2-{7-[4-({8-[2-(2-Furoylamino)éthyl]-2-naphtyl}oxy)butoxy]-1-naphtyl}éthyl)-2-furamide

Dans une fiole de 250 ml, mettre en suspension le composé obtenu au stade B (10 mmol) dans un mélange chloroforme/eau (3/2). Ajouter le carbonate de potassium (50 mmol) sous agitation. Refroidir le milieu réactionnel à 0°C à l'aide d'un bain de glace, puis ajouter le chlorure de 2-furoyle (22 mmol). Maintenir l'agitation à 0°C pendant 1 heure à température ambiante. Séparer les deux phases. Laver la phase organique avec une solution aqueuse d'acide chlorhydrique 1M puis à l'eau jusqu'à neutralité des eaux de lavage, la sécher sur du sulfate de magnésium et l'évaporer sous pression réduite. Recristalliser le résidu obtenu dans l'acétonitrile.
Point de fusion : 179-180°C

### Exemple 37 : 2-Bromo-N-[2-(7-{4-[(8-{2-[(bromoacétyl)amino]éthyl}-2-naphtyl)oxy] butoxy}-1-naphtyl)éthyl]acétamide

On procède comme dans l'Exemple 36 en remplaçant le chlorure de 2-furoyle au stade C par le chlorure de bromoacétyle.
Point de fusion : 155-157°C

### Exemple 38 : N-[2-(7-{4-[(8-{2-[(Cyclopropylcarbonyl)amino]éthyl}-2-naphtyl)oxy] butoxy}-1-naphtyl)éthyl]cyclopropane carboxamide

On procède comme dans l'Exemple 36 en remplaçant le chlorure de 2-furoyle au stade C par le chlorure de cyclopropane carbonyle.
Point de fusion : 177-179°C

### Exemple 39 : N-(2-{7-[4-({8-[2-(3-Buténoylamino)éthyl]-2-naphtyl}oxy)butoxy]-1-naphtyl}éthyl)-3-buténamide

On procède comme dans l'Exemple 36 en remplaçant le chlorure de 2-furoyle au stade C par le chlorure de 3-buténoyle.
Point de fusion : 146-148°C

### Exemple 40 : N-(2-{5-[4-({3-[2-(Acétylamino)éthyl]-1-benzothién-5-yl}oxy)butoxy]-1-benzothién-3-yl}éthyl)acétamide

Dans un ballon de 250 ml, dissoudre le composé obtenu dans la Préparation 33 dans l'acétonitrile, ajouter le carbonate de potassium et porter à reflux pendant 30 minutes. Ajouter par fractions le 1,4-dibromobutane, chauffer à reflux pendant 15 heures. Evaporer à sec, ajouter de l'eau, filtrer et recristalliser le précipité obtenu dans le dioxane.
Point de fusion : 202-204°C

### Exemple 41 : N-[2-(7-{[8-({8-[2-(Acétylamino)éthyl]-2-naphtyl}oxy)octyl]oxy}-1-naphtyl)éthyl]acétamide

Dans un ballon de 100 ml, dissoudre le composé obtenu dans la Préparation 1 (1,14 g, 5 mmol) dans 50 ml d'acétonitrile. Ajouter le carbonate de potassium (0,83 g, 6 mmol) et laisser sous agitation magnétique à reflux pendant 30 minutes. Ajouter ensuite goutte à goutte le 1,8-dibromooctane (2 mmol) (0,25 ml, 2 mmol) et chauffer à reflux pendant 12 heures. Evaporer sous pression réduite l'acétonitrile. Reprendre le résidu obtenu par une solution aqueuse de soude 1M. Filtrer le précipité obtenu, le laver à l'eau et le recristalliser dans l'acétonitrile.
Point de fusion : 137-139°C

### Exemple 42 : N-[2-(7-{[10-({8-[2-(Acétylamino)éthyl]-2-naphtyl}oxy}décyl]oxy}-1-naphtyl)éthyl]acétamide

On procède comme dans l'Exemple 40 en remplaçant le 1,8-dibromooctane par le 1,10-dibromodécane.
Point de fusion : 134-135°c

### Exemple 43 : {7-[4-({8-[2-(Acétylamino)éthyl]-2-naphtyl}oxy)butoxy]-1-naphtyl} acétate de méthyle

### Stade A : Acide 2-(7-hydroxynapht-1-yl)acétique

Dans un ballon de 250 mL, dissoudre l'acide 2-(7-méthoxy napht-1-yl)acétique dans l'acide acétique. Ajouter l'acide bromhydrique et porter le milieu réactionnel à reflux pendant 4 heures. Evaporer l'acide acétique et l'acide bromhydrique sous pression réduite. Reprendre le solide par l'eau et l'essorer. Laver le précipité à l'éther de pétrole et le recristalliser dans du toluène.
Point de fusion : 151-152°C

### Stade B : 2-(7-Hydroxynapht-1-yl)éthanoate de méthyle

Dissoudre le composé obtenu au stade A dans 100 mL de méthanol. Refroidir le mélange dans un bain de glace. Ajouter le chlorure de thionyle goutte à goutte et maintenir le milieu réactionnel à cette température pendant 20 minutes après l'addition. Laisser 1 heure à température ambiante sous agitation. Evaporer le méthanol sous pression réduite, et recristalliser le solide obtenu dans un mélange toluène-cyclohexane (4/1).
Point de fusion : 115°C

### Stade C : {7-[4-({8-[2-(Acétylamino)éthyl]-2-naphtyl}oxy)butoxy]-1-naphtyl}acétate de méthyle

Dans un ballon de 250 mL, dissoudre le composé dans l'acétonitrile, ajouter le carbonate de potassium, porter le milieu réactionnel à reflux pendant 30 minutes. Ajouter le dérivé bromé et maintenir le chauffage à reflux pendant 12 heures. Le milieu réactionnel est essoré. Le filtrat est évaporé sous pression réduite et l'huile obtenue est reprise dans l'éther sous agitation. Essorer et recristalliser le précipité obtenu dans le méthanol.
Point de fusion : 109-110°C

### Exemple 44 : Acide {7-[4-({8-[2-(acétylamino)éthyl]-2-naphtyl}oxy)butoxy]-1-naphtyl}acétique

Dans un ballon de 100 mL, dissoudre le composé obtenu dans l'Exemple 43 dans le THF, ajouter le méthanol, l'eau et la soude. Le milieu réactionnel est maintenu sous agitation à température ambiante pendant 4 heures. Concentrer la solution, hydrolyser et acidifier avec de l'acide chlorhydrique concentré. Essorer le précipité obtenu et recristalliser dans un mélange toluène/cyclohexane (4/1).
Point de fusion : 131-132°C

### Exemple 45 : N-(2-{7-[4-({8-[2-(Acétylamino)éthyl]-7-méthoxy-2-naphtyl}oxy) butoxy]-2-méthoxy-1-naphtyl}éthyl)acétamide

On procède comme dans l'Exemple 34 à partir du composé obtenu dans la Préparation 35.

### Exemple 46 : N-(2-{5-[4-({3-[2-(Acétylamino)éthyl]-1-méthyl-1H-pyrrolo[3,2-b] pyridin-5-yl}oxy)butoxy]-1-méthyl-1H-pyrrolo[3,2-b]pyridin-3-yl} éthyl)acétamide

On procède comme dans l'Exemple 34 à partir du composé obtenu dans la Préparation 32.

### Exemple 47 : N-(2-{5-[4-({3-[2-(Acétylamino)éthyl]-1-méthyl-1H-pyrrolo[3,2-b] pyridin-5-yl}oxy)propoxy]-1-méthyl-1H-pyrrolo[3,2-b]pyridin-3-yl} éthyl)acétamide

On procède comme dans l'Exemple 34 à partir du composé obtenu dans la Préparation 32 et en remplaçant le 1,4-dibromobutane par le 1,3-dibromopropane.

### Exemple 48 : N-{2-[5-{3-[2-(Acétylamino)éthyl]-1H-indol-3-yl}-1H-indol-3-yl]éthyl} acétamide

On procède comme dans l'Exemple 25 en remplaçant le composé obtenu dans la Préparation 24 par le composé obtenu dans la Préparation 33.
Point de fusion : 237-238°C

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la *pars tuberalis* de mouton. La *pars tuberalis* de l'adénohypophyse est en effet caractérisée, chez les mammifères, par une haute densité en récepteurs de la mélatonine (Journal of Neuroendocrinology, 1, pp. 1-4, 1989).

### Protocole

1) Les membranes de *pars tuberalis* de mouton sont préparées et utilisées comme tissu cible dans des expériences de saturation pour déterminer les capacités et affinités de liaison pour la 2-[¹²⁵I]-iodomélatonine.
2) Les membranes de *pars tuberalis* de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : Etude de liaison aux récepteurs mt₁ et MT₂ de la mélatonine

Les expériences de liaison aux récepteurs mt₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention attestent d'une liaison pour l'un ou l'autre des sous-types réceptoriels mt₁ ou MT₂, ces valeurs étant ≤ 10 µM.

### EXEMPLE D : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien *via* le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.

Après sacrifice, l'artère caudale est isolée et maintenue dans un milieu richement oxygéné. Les artères sont ensuite cannulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.

L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

1000 comprimés dosés à 5 mg de *N*-(2-{7-[4-({8-[2-(acétylamino)éthyl]-2-naphtyl} oxy)butoxy]-1-naphtyl}éthyl)acétamide (Exemple 12) 5 g
Amidon de blé 20 g
Amidon de maïs 20 g
Lactose 30 g
Stéarate de magnésium 2 g
Silice 1 g
Hydroxypropylcellulose 2 g

## Revendications

1. Composés de formule (I) :
A-G₁-Cy-G₂-Cy-G₃-B (I)
dans laquelle :
◆ A représente un groupement de formule 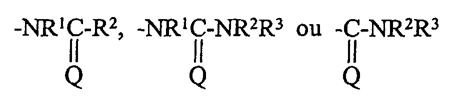 dans lesquelles :
- Q représente un atome de soufre ou d'oxygène,
- R¹, R², R³, identiques ou différents, représentent un atome d'hydrogène ou un groupement Rₐ [où Rₐ représente un groupement alkyle (C₁-C₆) linéaire ou ramifié non substitué ou substitué, alkényle (C₂-C₆) linéaire ou ramifié non substitué ou substitué, alkynyle (C₂-C₆) linéaire ou ramifié non substitué ou substitué, cycloalkyle (C₃-C₈) non substitué ou substitué, cycloalkyl (C₃-C₈) alkyle (C₁-C₆) linéaire ou ramifié non substitué ou substitué, polyhalogénoalkyle (C₁-C₆) linéaire ou ramifié, aryle, arylalkyle (dans lequel la partie alkyle contient 1 à 6 atomes de carbone et peut être linéaire ou ramifiée), arylalkényle (dans lequel la partie alkényle contient 2 à 6 atomes de carbone et peut être linéaire ou ramifiée), hétéroaryle, hétéroarylalkyle (dans lequel la partie alkyle contient 1 à 6 atomes de carbone et peut être linéaire ou ramifiée) ou hétéroarylalkényle (dans lequel la partie alkényle contient 2 à 6 atomes de carbone et peut être linéaire ou ramifiée)],
ou les groupements R² et R³ forment, avec l'atome d'azote qui les porte un groupement choisi parmi piperazinyle, pipéridinyle, ou pyrrolidinyle,
◆ B représente un groupement de formule 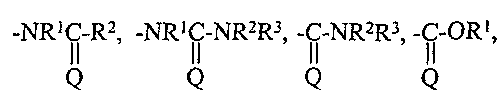 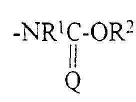 ou -NR²R³ où Q, R¹, R² et R³ sont tels que définis précédemment,
◆ G₁ et G₃, identiques ou différents, représentent une chaîne alkylène contenant de 1 à 4 carbones linéaire ou ramifiée éventuellement substituée par un ou plusieurs groupements, identiques ou différents, choisis parmi hydroxy, carboxy, formyle, Rₐ, ORₐ, COORₐ ou CORₐ (où Rₐ est tel que défini précédemment),
◆ Cy représente
- une structure cyclique de formule (II) : 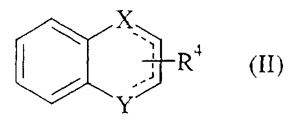 dans laquelle :
∗ X et Y, identiques ou différents, représentent un atome de soufre, d'oxygène ou de carbone, ou un groupement CH ou CH₂,
∗ R⁴ représente un atome d'hydrogène ou d'halogène ou un groupement CF₃, hydroxy, carboxy, formyle, amino, NHRₐ, NRₐR¹ₐ, NHCORₐ, CONHRₐ, Rₐ, ORₐ, CORₐ ou COORₐ (où Rₐ est tel que défini précédemment et R¹ₐ peut prendre toutes les valeurs de Rₐ).
∗ la représentation ---- signifie que les liaisons sont simples ou doubles, étant entendu que la valence des atomes est respectée,
où G₂ substitue le cycle benzénique, et G₁ (G₃ respectivement) substitue le cycle contenant X et Y,
- ou une structure cyclique de formule (III) : 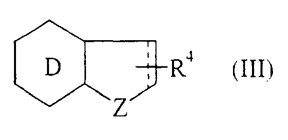 dans laquelle :
∗ Z représente un atome de soufre ou d'oxygène, ou un groupement CH, CH₂, NH, NSO₂Ph ou NRₐ (où Rₐ est tel que défini précédemment),
∗ D représente un cycle benzénique ou pyridinique,
∗ R⁴ est tel que défini précédemment,
∗ la représentation ---- signifie que la liaison est simple ou double, étant entendu que la valence des atomes est respectée,
où G₂ substitue le cycle D, et G₁ (G₃ respectivement) substitue le cycle contenant Z,
étant entendu que les deux cycles (Cy) des composés de formule (I) représentent la même structure cyclique de base (indole/indole, naphtalène/naphtalène, benzofurane/ benzofurane etc...), mais le substituant R⁴ peut être différent,
◆ G₂ représente une chaîne de formule (IV) : 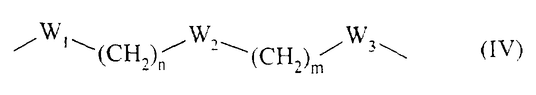 dans laquelle :
- W₁, W₂ et W₃, identiques ou différents, représentent une liaison, un atome d'oxygène ou de soufre, ou un groupement CH₂, CHRₐ, NH ou NRₐ (où Rₐ est tel que défini précédemment),
- n représente un entier tel que 0 ≤ n ≤ 6,
- m représente un entier tel que 0 ≤ m ≤ 6,
étant entendu que l'on ne peut pas avoir deux hétéroatomes consécutifs, et que la chaîne de formule (IV) ainsi définie peut comporter une ou plusieurs insaturations,
étant entendu que :
- le composé de formule (I) ne peut représenter le 2-(acétylamino)-2-{[5-({3-[2-(acétylamino)-3-éthoxy-2-(éthoxycarbonyl)-3-oxopropyl]-*1H*-indol-5-yl}méthyl) *1H*-indol-3-yl]méthyl}malonate de diéthyle, le 2-(acétylamino)-2-{[5-{[3-[2-(acétylamino)-3-éthoxy-2-(éthoxycarbonyl)-3-oxopropyl]-2-(éthoxycarbonyl)-1*H*-indol-5-yl]méthyl}-2-(éthoxycarbonyl)-1*H*-indol-3-yl]méthyl}malonate de diéthyle, ni le N-{2-[5-({3-[2-(acétylamino)éthyl]-*1H*-indol-5-yl}méthyl)-*1H*-indol-3-yl]éthyl}acétamide,
- par « aryle », on entend les groupements naphtyle, phényle et biphényle,
- par « hétéroaryle », on entend tout groupement mono ou bicyclique, saturé ou insaturé contenant 5 à 10 atomes et contenant 1 à 3 hétéroatomes choisis parmi azote, soufre et oxygène,
les groupements « aryles» et « hétéroaryles» pouvant être substitués par un ou plusieurs radicaux, identiques ou différents, choisis parmi hydroxy, carboxy, alkoxy (C₁-C₆) linéaire ou ramifié, alkyle (C₁-C₆) linéaire ou ramifié, polyhalogénoalkyle(C₁-C₆) (C₁-C₆) linéaire ou ramifié, formyle, cyano, nitro, amino, alkylamino (C₁-C₆) linéaire ou ramifié, dialkylamino (C₁-C₆) linéaire ou ramifié, ou atomes d'halogène,
- le terme « substitué » affecté aux expressions « alkyle », « alkényle », et « alkynyle » signifie que ces groupements sont substitués par un ou plusieurs radicaux, identiques ou différents, choisis parmi hydroxy, alkoxy (C₁-C₆) linéaire ou ramifié, polyhalogénoalkyle (C₁-C₆) linéaire ou ramifié, amino, alkylamino (C₁-C₆) linéaire ou ramifié, dialkylamino (C₁-C₆) linéaire ou ramifié, ou atomes d'halogène,
- le terme « substitué » affecté aux expressions « cycloalkyle » et « cycloalkylalkyle » signifie que la partie cyclique de ces groupements est substituée par un ou plusieurs radicaux, identiques ou différents, choisis parmi hydroxy, alkoxy (C₁-C₆) linéaire ou ramifié, polyhalogénoalkyle (C₁-C₆) linéaire ou ramifié, amino. alkylamino (C₁-C₆) linéaire ou ramifié, dialkylamino (C₁-C₆) linéaire ou ramifié, ou atomes d'halogène.
leurs énantiomères et diastéréoisomères, ainsi que leurs sels d'addition à un acide ou une base pharmaceutiquement acceptable.

2. Composés de formule (I) selon la revendication 1 pour lesquels Cy représente une structure cyclique de formule (II), leurs énantiomères et diastéréoisomères. ainsi que leurs sels d'addition à un acide ou une base pharmaceutiquement acceptable.

3. Composés de formule (I) selon la revendication 1 pour lesquels Cy représente un naphtalène, leurs énantiomères et diastéréoisomères, ainsi que leurs sels d'addition à un acide ou une base pharmaceutiquement acceptable.

4. Composés de formule (I) selon la revendication 1 pour lesquels Cy représente une structure cyclique de formule (III), leurs énantiomères et diastéréoisomères, ainsi que leurs sels d'addition à un acide ou une base pharmaceutiquement acceptable.

5. Composés de formule (I) selon la revendication 1 pour lesquels Cy représente un benzothiophène ou un benzofurane, leurs énantiomères et diastéréoisomères, ainsi que leurs sels d'addition à un acide ou une base pharmaceutiquement acceptable.

6. Composés de formule (I) selon la revendication 1 pour lesquels Cy représente un indole, leurs énantiomères et diastéréoisomères, ainsi que leurs sels d'addition à un acide ou une base pharmaceutiquement acceptable.

7. Composés de formule (I) selon la revendication 1 pour lesquels Cy représente un azaindole, leurs énantiomères et diastéréoisomères, ainsi que leurs sels d'addition à un acide ou une base pharmaceutiquement acceptable.

8. Composés de formule (I) selon la revendication 1 pour lesquels G₂ représente une liaison simple, leurs énantiomères et diastéréoisomères, ainsi que leurs sels d'addition à un acide ou une base pharmaceutiquement acceptable.

9. Composés de formule (I) selon la revendication 1 pour lesquels G₂ représente un groupement -W₄-(CH₂)ₚ-W'₄- dans lequel W₄ et W'₄, identiques ou différents, représentent un atome d'oxygène ou de soufre ou un groupement NH ou NRₐ, et p représente un entier tel que 1 ≤ p ≤ 12, leurs énantiomères et diastéréoisomères, ainsi que leurs sels d'addition à un acide ou une base pharmaceutiquement acceptable.

10. Composés de formule (I) selon la revendication 1 pour lesquels G₂ représente un groupement -O-(CH₂)ₚ-O- dans lequel p représente un entier tel que 1 ≤ p ≤ 12, leurs énantiomères et diastéréoisomères. ainsi que leurs sels d'addition à un acide ou une base pharmaceutiquement acceptable.

11. Composés de formule (I) selon la revendication 1 pour lesquels G₂ représente un groupement W₄-(CH₂)_{p'}-W'₄-(CH₂)_{p"}-W"₄- dans lequel W₄, W'₄ et W"₄, identiques ou différents, représentent un atome d'oxygène ou de soufre ou un groupement NH ou NRₐ, et p' et p" sont des entiers tels que 2 ≤ p' + p" ≤ 12, leurs énantiomères et diastéréoisomères, ainsi que leurs sels d'addition à un acide ou une hase pharmaceutiquement acceptable.

12. Composés de formule (I) selon la revendication 1 pour lesquels G₂ représente un groupement -O-(CH₂)_{p'}-O-(CH₂)_{p"}-O- (où p' et p" sont tels que définis précédemment), leurs énantiomères et diastéréoisomères, ainsi que leurs sels d'addition à un acide ou une base pharmaceutiquement acceptable.

13. Composés de formule (I) selon la revendication 1 pour lesquels A et B, identiques ou différents, représentent un groupement NR¹COR² ou CONR²R³, leurs énantiomères et diastéréoisomères, ainsi que leurs sels d'addition à un acide ou une base pharmaceutiquement acceptable.

14. Composés de formule (I) selon la revendication 1 qui sont le *N*-(2-{7-[2-({8-[2-(acétylamino)éthyl]-2-naphtyl}oxy)éthoxy]-1-naphtyl} éthyl)acétamide, le *N*-(2-{7-[3-({8-[2-(acétylamino)éthyl]-2-naphtyl}oxy)propoxy]-1-naphtyl}éthyl)acétamide, le *N*-(2-{7-[4-({8-[2-(acétylamino)éthyl]-2-naphtyl}oxy)butoxy]-1-naphtyl}éthyl)acétamide, le *N*-[2-(7-{[6-({8-[2-(acétylamino)éthyl]-2-naphtyl}oxy)hexyl]oxy}-1-naphtyl)éthyl]acétamide, le *N*-[2-(7-{[5-({8-[2-(acétylamino)éthyl]-2-naphtyl}oxy)pentyl]oxy}-1-naphtyl)éthyl]acétamide, le *N*-[2-(7-{[8-({8-[2-(acétylamino)éthyl]-2-naphtyl}oxy)octyl]oxy}-1-naphtyl)éthyl] acétamide, le *N*-[2-(7-{[10-({8-[2-(acétylamino)éthyl]-2-naphtyl}oxy)décyl]oxy}-1-naphtyl) éthyl]acétamide et le *N*-(2-{7-[4-({8-[2-(acétylamino)éthyl]-7-méthoxy-2-naphtyl}oxy)butoxy]-2-méthoxy-1-naphtyl}éthyl)acétamide, ainsi que leurs sels d'addition à un acide ou une base pharmaceutiquement acceptable.

15. Composé de formule (I) selon la revendication 1 qui est le *N*-[2-(7-{2-[2-({8-[2-(acétylamino)éthyl]-2-naphtyl}oxy)éthoxy]éthoxy}-1-naphtyl)éthyl]acétamide ainsi que ses sels d'addition à un acide ou une base pharmaceutiquement acceptable.

16. Composés de formule (I) selon la revendication 1 qui sont le *N*-(2-{7-[4-({8-[2-(2-furoylamino)éthyl]-2-naphtyl}oxy)butoxy]-1-naphtyl}éthyl)-2-furamide, le 2-bromo-*N*-[2-(7-{4-[(8-{2-[(bromoacétyl)amino]éthyl}-2-naphtyl)oxy]butoxy}-1-naphtyl)éthyl]acétamide, le *N*-[2-(7-{4-[(8-{2-[(cyclopropylcarbonyl)amino]éthyl}-2-naphtyl)oxy]butoxy}-1-naphtyl) éthyl]cyclopropane carboxamide et le *N*-(2-{7-[4-({8-[2-(3-buténoylamino)éthyl]-2-naphtyl}oxy)butoxy]-1-naphtyl}éthyl)-3-buténamide, ainsi que leurs sels d'addition à un acide ou une base pharmaceutiquement acceptable.

17. Composé de formule (I) selon la revendication 1 qui est le *N*-(2-{7-[4-({8-[2-(acétylamino)éthyl]-5,6,7,8-tétrahydro-2-naphtalényl}oxy)butoxy]-1,2,3,4-tétrahydro-1-naphtalényl}éthyl)acétamide, ses énantiomères et diastéréoisomères, ainsi que ses sels d'addition à un acide ou une base pharmaceutiquement acceptable.

18. Composés de formule (I) selon la revendication 1 qui sont le *N*-{2-[5-(4-{[3-[2-(acétylamino)éthyl]-1-(phénylsulfonyl)-*1H*-indol-5-yl]oxy}butoxy)-1-(phénylsulfonyl)-*1H*-indol-3-yl]éthyl}acétamide et le *N*-(2-{5-[4-({3-[2-(acétylamino)éthyl]-*1H*-indol-5-yl}oxy)butoxy]-*1H*-indol-3-yl}éthyl)acétamide, ainsi que leurs sels d'addition à un acide ou une base pharmaceutiquement acceptable.

19. Composés de formule (I) selon la revendication 1 qui sont le *N*-(2-{5-[4-({3-[2-(acétylamino)éthyl]-1-benzofuran-5-yl}oxy)butoxy]-1-benzofuran-3-yl}éthyl)acétamide et le *N*-(2-{5-[4-({3-[2-(acétylamino)éthyl]-1-benzothién-5-yl}oxy)butoxy]-1-benzothién-3-yl}éthyl)acétamide, ainsi que leurs sels d'addition à un acide ou une base pharmaceutiquement acceptable.

20. Composés de formule (I) selon la revendication 1 qui sont le *N*-(2-{5-[4-({3-[2-(acétylamino)éthyl]-1-méthyl-*1H*-pyrrolo*[3,2-b]*pyridin-5-yl}oxy)butoxy]-1-méthyl-*1H*-pyrrolo*[3,2-b]*pyridin-3-yl}éthyl)acétamide et le *N*-(2-{5-[4-({3-[2-(acétylamino)éthyl]-1-méthyl-*1H*-pyrrolo*[3,2-b]*pyridin-5-yl}oxy)propoxy]-1-méthyl-*1H*-pyrrolo*[3,2-b]*pyridin-3-yl}éthyl)acétamide, ainsi que leurs sels d'addition à un acide ou une base pharmaceutiquement acceptable.

21. Composé de formule (I) selon la revendication 1 qui est le *N*-[2-(7-{8-[2-(acétylamino)éthyl]-2-naphtyl}-1-naphtyl)éthyl]acétamide, ainsi que ses sels d'addition à un acide ou une base pharmaceutiquement acceptable.

22. Composé de formule (I) selon la revendication 1 qui est le *N*-{2-[5-{3-[2-(acétylamino)éthyl]-*1H*-indol-3-yl}-*1H*-indol-3-yl]éthyl}acétamide, ainsi que ses sels d'addition à un acide ou une base pharmaceutiquement acceptable.

23. Procédé de préparation des composés de formule (I) selon la revendication 1 **caractérisé en ce que** l'on utilise comme produit de départ le composé de formule (V) :
A-G₁-Cy-OMe (V)
dans laquelle A, G₁ et Cy sont tels que définis dans la formule (I),
que l'on soumet à une déméthylation en utilisant des agents classiques comme HBr, AlCl₃, AlBr₃, BBr₃ ou des systèmes binaires acide de Lewis / nucléophile comme AlCl₃ / PhCH₂SH ou BBr₃ / Me₂S par exemple, pour obtenir le composé de formule (VI) :
A-G₁-Cy-OH (VI)
dans laquelle A, G₁ et Cy sont définis comme précédemment,
◆ qui est transformé, de façon classique,
- par action de *N,N*-diméthylthiocarbamate de sodium par exemple, en thiol correspondant de formule (VII) :
A-G₁-Cy-SH (VII)
dans laquelle A, G₁ et Cy sont tels que définis précédemment,
- ou en dérivé aminé correspondant de formule (VIII) :
A-G₁-Cy-NHR'ₐ (VIII)
dans laquelle A, G₁ et Cy sont définis comme précédemment et R'ₐ peut prendre toutes les valeurs de Rₐ tel que défini dans la formule (I) et peut également représenter un atome d'hydrogène,
les composés de formule (VI), (VII) et (VIII) représentant le composé de formule (IX) :
A-G₁-Cy-W₄H (IX)
dans laquelle W₄ représente un atome d'oxygène ou de soufre, ou un groupement NH ou NRₐ (où Rₐ est défini comme précédemment),
composé de formule (IX) sur lequel on condense
• un composé de formule (X) : 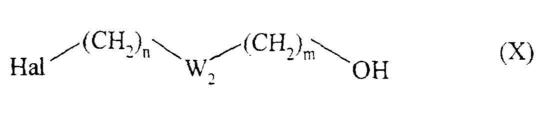 dans laquelle Hal représente un atome de brome, de chlore ou d'iode, et n, W₂ et m sont tels que définis dans la formule (I), (étant entendu que l'on ne peut pas avoir deux hétéroatomes consécutifs et que la chaîne ainsi définie peut comporter une ou plusieurs insaturations),
• ou un composé de formule (XI) : 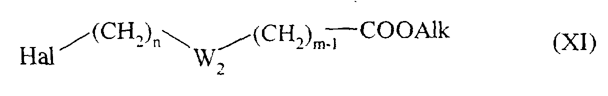
dans laquelle Hal, W₂, n et m sont définis comme précédemment et Alk représente un radical alkyle (étant entendu que l'on ne peut pas avoir deux hétéroatomes consécutifs et que la chaîne ainsi définie peut comporter une ou plusieurs insaturations), suivi d'une réduction,
pour conduire au composé de formule (XII) :
A-G₁-Cy-W₄-(CH₂)ₙ-W₂-(CH₂)ₘ-OH (XII)
dans laquelle A, G₁, Cy, W₂, W₄, n et m sont définis comme dans la formule (I) (étant entendu que l'on ne peut pas avoir deux hétéroatomes consécutifs dans l'enchaînement W₄-(CH₂)ₙ-W₂-(CH₂)ₘ-OH et que la chaîne ainsi définie peut comporter une ou plusieurs insaturations),
dont la fonction hydroxyle est transformée classiquement en groupe partant comme par exemple un mésylate, un tosylate, ou un dérivé halogéné, pour conduire au composé de formule (XII') :
A-G₁-Cy-W₄-(CH₂)ₙ-W₂-(CH₂)ₘ-E (XII')
dans laquelle A, G₁, Cy, W₄, n, W₂ et m sont définis comme précédemment et E représente un groupement mésyle ou tosyle ou un atome d'halogène,
sur lequel on fait agir un composé de formule (XIII) :
B-G₃-Cy-W'₄H (XIII)
dans laquelle B, G₃ et Cy sont définis comme dans la formule (I) et W'₄ peut prendre les mêmes valeurs que W₄ défini précédemment,
pour conduire au composé de formule (I/a), cas particulier des composés de formule (I) :
A-G₁-Cy-W₄-(CH₂)ₙ-W₂-(CH₂)ₘ-W'₄-Cy-G₃-B (I/a)
dans laquelle A. G₁, Cy, W₄, n, W₂, m, W'₄, G₃ et B sont tels que définis précédemment,
(les composés de formule (I/a) pour lesquels les groupements A-G₁-Cy-W₄- et W'₄-Cy-G₃-B sont identiques pouvant être obtenus directement à partir du composé de formule (IX) sur lequel on condense en milieu basique un composé de formule (X') : 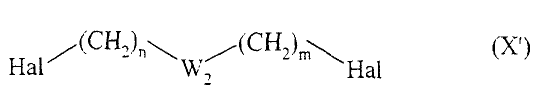 dans laquelle Hal, n, m et W₂ ont la même définition que précédemment),
◆ ou transformé en utilisant par exemple le phényl bis (trifluorométhane-sulfonimide) en milieu basique en trifluorométhane sulfonate correspondant de formule (XIV) :
A-G₁-Cy-OSO₂CF₃ (XIV)
dans laquelle A, G₁ et Cy sont définis comme précédemment,
- sur lequel on fait agir, dans des conditions de catalyse par un dérivé du palladium approprié, un dérivé de l'acide borique (R_{b}B(OH)₂) ou un dérivé de l'étain (R_{b}SnBu₃) (où R_{b} représente un groupement de formule (XV) :
B-G₃-Cy-W₃-(CH₂)ₘ-W₂-(CH₂)ₙ-CH₂- (XV)
dans laquelle B, G₃, Cy, W₃, m, W₂ et n sont tels que définis précédemment, étant entendu que l'on ne peut pas avoir deux hétéroatomes consécutifs dans l'enchaînement -W₃-(CH₂)ₘ-W₂- et que la chaîne ainsi définie peut comporter une ou plusieurs insaturations),
pour conduire au composé de formule (I/b), cas particulier des composés de formule (I) :
A-G₁-Cy-CH₂-(CH₂)ₙ-W₂-(CH₂)ₘ-W₃-Cy-G₃-B (I/b)
dans laquelle A, G₁, Cy, n, W₂, m, W₃, Cy, G₃ et B sont définis comme précédemment (étant entendu que l'on en peut pas avoir deux hétéroatomes consécutifs dans l'enchaînement -W₂-(CH₂)ₘ-W₃- et que la chaîne ainsi définie peut comporter une ou plusieurs insaturations),
les composés de formule (I/c), cas particulier des composés de formule (I) :
A-G₁-Cy-W₁-(CH₂)ₙ-W₂-(CH₂)ₘ-CH₂-Cy-G₃-B (I/c)
dans laquelle A, G₁, Cy, W₁, n, W₂, m, G₃ et B sont définis comme précédemment (étant entendu que l'on ne peut pas avoir deux hétéroatomes consécutifs dans l'enchaînement -W₁-(CH₂)ₙ-W₂- et que la chaîne ainsi définie peut comporter une ou plusieurs insaturations),
étant obtenus selon un mode opératoire similaire à partir du composé de formule (XIV') :
B-G₃-Cy-OSO₂CF₃ (XIV')
dans laquelle B, G₃ et Cy sont tels que définis précédemment,
- ou que l'on place, dans des conditions de couplage en utilisant des dérivés du Nickel ou du palladium par exemple, en présence d'un composé de formule (XIV') afin de conduire au composé de formule (I/d), cas particulier des composés de formule (I)
A-G₁-Cy-Cy-G₃-B (I/d)
dans laquelle A, G₁, Cy, G₃ et B sont tels que définis précédemment,
l'ensemble des composés (I/a) à (I/d) formant le composé de formule (I) qui peut être purifié si on le désire par une technique classique de purification, dont on sépare éventuellement les isomères selon une technique classique de séparation et que l'on transforme, le cas échéant, en leurs sels d'addition à un acide ou à une base pharmaceutiquement acceptable.

24. Composés de formule (XII'') :
A-G₁-Cy-W₄-(CH₂)ₙ-W₂-(CH₂)ₘ-E' (XIIʺ)
dans laquelle :
◆ A représente un groupement de formule 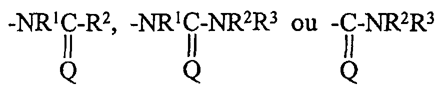 dans lesquelles :
- Q représente un atome de soufre ou d'oxygène,
- R¹, R², R³, identiques ou différents, représentent un atome d'hydrogène ou un groupement Rₐ [où Rₐ représente un groupement alkyle (C₁-C₆) linéaire ou ramifié non substitué ou substitué, alkényle (C₂-C₆) linéaire ou ramifié non substitué ou substitué, alkynyle (C₂-C₆) linéaire ou ramifié non substitué ou substitué, cycloalkyle (C₃-C₈) non substitué ou substitué, cycloalkyl (C₃-C₈) alkyle (C₁-C₆) linéaire ou ramifié non substitué ou substitué, polyhalogénoalkyle (C₁-C₆) linéaire ou ramifié, aryle, arylalkyle (dans lequel la partie alkyle contient 1 à 6 atomes de carbone et peut être linéaire ou ramifiée), arylalkényle (dans lequel la partie alkényle contient 2 à 6 atomes de carbone et peut être linéaire ou ramifiée), hétéroaryle, hétéroarylalkyle (dans lequel la partie alkyle contient 1 à 6 atomes de carbone et peut être linéaire ou ramifiée) ou hétéroarylalkényle (dans laquelle la partie alkényle contient 2 à 6 atomes de carbone et peut être linéaire ou ramifiée)],
ou les groupements R² et R³ forment, avec l'atome d'azote qui les porte un groupement choisi parmi piperazinyle, pipéridinyle, ou pyrrolidinyle,
◆ G₁ représente une chaîne alkylène contenant de 1 à 4 carbones linéaire ou ramifiée éventuellement substituée par un ou plusieurs groupements, identiques ou différents, choisis parmi hydroxy, carboxy, formyle, Rₐ, ORₐ, COORₐ ou CORₐ (où Rₐ est tel que défini précédemment),
◆ Cy représente
- une structure cyclique de formule (II) : 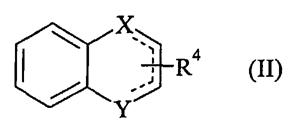 dans laquelle :
∗ X et Y, identiques ou différents, représentent un atome de soufre, d'oxygène ou de carbone, ou un groupement CH ou CH₂,
∗ R⁴ représente un atome d'hydrogène ou d'halogène ou un groupement CF₃, hydroxy, carboxy, formyle, amino, NHRₐ, NRₐR¹ₐ, NHCORₐ, CONHRₐ, Rₐ, ORₐ, CORₐ ou COORₐ (où Rₐ est tel que défini précédemment et R¹ₐ peut prendre toutes les valeurs de Rₐ),
∗ la représentation ---- signifie que les liaisons sont simples ou doubles, étant entendu que la valence des atomes est respectée,
où G₁ substitue le cycle contenant X et Y,
- ou une structure cyclique de formule (III) : 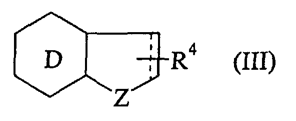 dans laquelle :
∗ Z représente un atome de soufre ou d'oxygène, ou un groupement CH, CH₂, NH, NSO₂Ph ou NRₐ (où Rₐ est tel que défini précédemment),
∗ D représente un cycle benzénique ou pyridinique,
∗ R⁴ est tel que défini précédemment,
∗ la représentation ---- signifie que la liaison est simple ou double, étant entendu que la valence des atomes est respectée,
où G₁ substitue le cycle contenant Z,
◆ W₂ représente une liaison, un atome d'oxygène ou de soufre, ou un groupement CH₂, CHRₐ, NH ou NRₐ (où Rₐ est tel que défini précédemment),
◆ W₄ représente un atome d'oxygène ou de soufre, ou un groupement NH ou NRₐ (où Rₐ est défini comme précédemment),
◆ n représente un entier tel que 0 ≤ n ≤ 6,
◆ m représente un entier tel que 0 ≤ m ≤ 6,
étant entendu que l'on ne peut pas avoir deux hétéroatomes consécutifs, et que la chaîne -W₄-(CH₂)ₙ-W₂-(CH₂)ₘ- ainsi définie peut comporter une ou plusieurs insaturations,
◆ et E' représente un groupement hydroxyle ou un atome d'halogène (fluor, chlore, brome ou iode),
étant entendu que
- lorsque Cy représente un naphtalène et G₁-A représente un groupement -(CH₂)₂-NR¹C(Q)R² ou -(CH₂)₂-NR¹C(Q)NR²R³ (où R¹, R² et R³ sont tels que définis précédemment), alors la chaîne -W₄-(CH₂)ₙ-W₂-(CH₂)ₘ- ne peut représenter une chaîne -O-alkyle-,
- le composé de formule (XII") ne peut représenter le *N*-{2-[5-(2-hydroxyéthoxy)-*1H*-indol-3-yl]éthyl} acétamide,
leurs énantiomères et diastéréoisomères, ainsi que leurs sels d'addition à un acide ou une base pharmaceutiquement acceptable,
en tant qu'intermédiaires de synthèse mais également en tant que composés utiles pour le traitement des troubles liés au système mélatoninergique.

25. Compositions pharmaceutiques contenant comme principe actif au moins un composé de formule (I) selon l'une quelconque des revendications 1 à 22 ou 24 ou un de leurs sels d'addition avec un acide ou une base pharmaceutiquement acceptables en combinaison avec un ou plusieurs excipients pharmaceutiquement acceptables.

26. Compositions pharmaceutiques selon la revendication 25 utiles pour la fabrication d'un médicament pour traiter les troubles liés au système mélatoninergique.

## Patentansprüche

1. Verbindungen der Formel (I):
A-G₁-Cy-G₂-Cy-G₃-B (I)
in der:
◆ A eine Gruppe der Formeln 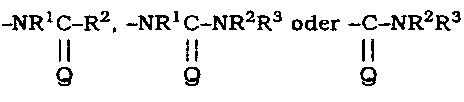 darstellt, in denen:
- Q ein Schwefel- oder Sauerstoffatom,
- R¹, R² und R³, die gleichartig oder verschieden sind, Wasserstoffatome oder eine Gruppe Rₐ darstellen [worin Rₐ eine geradkettige oder verzweigte, nichtsubstituierte oder substituierte (C₁-C₆)-Alkylgruppe, geradkettige oder verzweigte, nichtsubstituierte oder substituierte (C₂-C₆)-Alkenylgruppe, geradkettige oder verzweigte, nichtsubstituierte oder substituierte (C₂-C₆)-Alkinylgruppe, nichtsubstituierte oder substituierte (C₃-C₈)-Cycloalkylgruppe, geradkettige oder verzweigte, nichtsubstituierte oder substituierte (C₃-C₈)-Cycloalkyl-(C₁-C₆)-alkylgruppe, geradkettige oder verzweigte (C₁-C₆)-Polyhalogenalkylgruppe, Arylgruppe, Arylalkylgruppe (worin der Alkylrest 1 bis 6 Kohlenstoffatome enthält und geradkettig oder verzweigt sein kann), Arylalkenylgruppe (worin der Alkenylrest 2 bis 6 Kohlenstoffatome enthält und geradkettig oder verzweigt sein kann), Heteroarylgruppe, Heteroarylalkylgruppe (worin der Alkylrest 1 bis 6 Kohlenstoffatome enthält und geradkettig oder verzweigt sein kann) oder Heteroarylalkenylgruppe (worin der Alkenylrest 2 bis 6 Kohlenstoffatome enthält und geradkettig oder verzweigt sein kann) bedeutet],
oder die Gruppen R² und R³ zusammen mit dem sie tragenden Stickstoffatom eine Gruppe ausgewählt aus Piperazinyl, Piperidinyl oder Pyrrolidinyl bilden,
◆ B eine Gruppe der Formeln 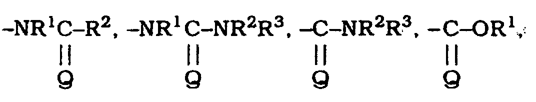 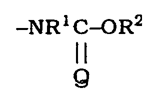 oder -NR²R³ darstellt, in denen Q, R¹, R² und R³ die oben angegebenen Bedeutungen besitzen.
◆ G₁ und G₃, die gleichartig oder verschieden sind, eine geradkettige oder verzweigte Alkylenkette bedeuten, die 1 bis 4 Kohlenstoffatome enthält und gegebenenfalls durch eine oder mehrere gleichartige oder verschiedenartige Gruppen ausgewählt aus Hydroxy, Carboxy, Formyl, Rₐ, ORₐ, COORₐ oder CORₐ (worin Rₐ die oben angegebenen Bedeutungen besitzt) substituiert sein kann,
◆ Cy
- eine cyclische Struktur der Formel (II): 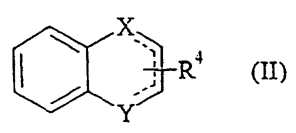 in der:
∗ X und Y, die gleichartig oder verschieden sind, ein Schwefelatom, ein Sauerstoffatom oder ein Kohlenstoffatom oder eine Gruppe CH oder CH₂ bedeuten,
∗ R⁴ ein Wasserstoffatom oder ein Halogenatom oder eine Gruppe CF₃, eine Hydroxygruppe, Carboxygruppe, Formylgruppe, Aminogruppe, eine Gruppe der Formeln NHRₐ, NRₐR¹ₐ, NHCORₐ, CONHRₐ, Rₐ, ORₐ, CORₐ oder COORₐ (worin Rₐ die oben angegebenen Bedeutungen besitzt und R¹ₐ sämtliche Bedeutungen von Rₐ annehmen kann) bedeutet,
∗ das Symbol ---- bedeutet, daß die Bindungen einfach oder doppelt sind, wobei es sich versteht, daß die Wertigkeit der Atome respektiert ist,
worin G₂ den Benzolring substituiert und G₁ (beziehungsweise G₃) den X und Y enthaltenden Ring substituiert,
- oder eine cyclische Struktur der Formel (III) bedeutet: 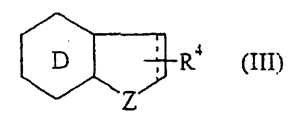 in der:
∗ Z ein Schwefelatom, ein Sauerstoffatom oder eine Gruppe CH, CH₂, NH, NSO₂Ph oder NRₐ (worin Rₐ die oben angegebenen Bedeutungen besitzt) darstellt,
∗ D einen Benzyl- oder Pyridin-Ring darstellt,
∗ R⁴ die oben angegebenen Bedeutungen besitzt,
∗ das Symbol ---- bedeutet, daß die Bindung einfach oder doppelt ist, wobei es sich versteht, daß die Wertigkeit der Atome respektiert ist,
worin G₂ den Ring D substituiert und G₁ (beziehungsweise G₃) den Z enthaltenden Ring substituiert,
wobei es sich versteht, daß die beiden Ringe (Cy) der Verbindungen der Formel (I) die gleiche cyclische Grundstruktur darstellen (Indol/Indol, Naphthalin/ Naphthalin, Benzofuran/Benzofuran, etc...), jedoch der Substituent R⁴ verschiedenartig sein kann,
◆ G₂ eine Kette der Formel (IV) darstellt: 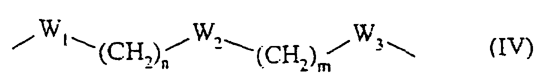 in der:
- W₁, W₂ und W₃, die gleichartig oder verschieden sind, eine Bindung, ein Sauerstoffatom oder ein Schwefelatom oder eine Gruppe CH₂, CHRₐ, NH oder NRₐ (worin Rₐ die oben angegebenen Bedeutungen besitzt), darstellen,
- n eine ganze Zahl darstellt, so daß die Beziehung 0 ≤ n ≤ 6 erfüllt ist,
- m eine ganze Zahl darstellt, so daß die Beziehung 0 ≤ m ≤ 6 erfüllt ist,
mit der Maßgabe, daß nicht zwei aufeinanderfolgende Heteroatome vorliegen und daß die in dieser Weise definierte Kette der Formel (IV) eine oder mehrere Unsättigungen aufweisen kann,
wobei es sich versteht, daß:
- die Verbindung der Formel (I) nicht 2-(Acetylamino)-2-{[5-({3-(2-(acetylamino)-3-ethoxy-2-(ethoxycarbonyl)-3-oxopropyl]-*1H*-indol-5-yl}-methyl)-*1H*indol-3-yl]-methyl}-malonsäurediethylester, 2-(Acetylamino)-2-{[5-{[3-[2-(acetylamino)-3-ethoxy-2-(ethoxycarbonyl)-3-oxopropyl]-2-(ethoxycarbonyl)-*1H*indol-5-yl]-methyl}-2-(ethoxycarbonyl)-*1H*-indol-3-yl]-methyl}-malonsäurediethylester noch N-{2-[5-({3-[2-(Acetylamino)-ethyl]-*1H*-indol-5-yl}-methyl)-*1H*-indol-3-yl]-ethyl}-acetamid bedeutet,
- unter «Aryl» Naphthyl-, Phenyl- und Biphenylgruppen zu verstehen sind,
- unter «Heteroaryl» jede mono- oder bicyclische, gesättigte oder ungesättigte Gruppe zu verstehen ist, die 5 bis 10 Atome aufweist und 1 bis 3 Heteroatome ausgewählt aus Stickstoff, Schwefel und Sauerstoff enthält, die «Aryl»- und «Heteroaryl»-gruppen durch eine oder mehrere gleichartige oder verschiedene Gruppen ausgewählt aus Hydroxy, Carboxy, geradkettigem oder verzweigtem (C₁ -C₆)-Alkoxy, geradkettigem oder verzweigtem (C₁-C₆)-Alkyl, geradkettigem oder verzweigtem (C₁-C₆)-Polyhalogenalkyl, Formyl, Cyano, Nitro, Amino, geradkettigem oder verzweigtem (C₁-C₆)-Alkylamino, geradkettigem oder verzweigtem (C₁-C₆)-Dialkylamino oder Halogenatomen substituiert sein können,
- der Begriff «substituiert», bezogen auf die Begriffe «Alkyl», «Alkenyl» und «Alkinyl» bedeutet, daß diese Gruppen durch eine oder mehrere gleichartige oder verschiedene Gruppen ausgewählt aus Hydroxy, geradkettigem oder verzweigtem (C₁-C₆)-Alkoxy, geradkettigem oder verzweigtem (C₁-C₆)-Polyhalogenalkyl, Amino, geradkettigem oder verzweigtem (C₁-C₆)-Alkylamino, geradkettigem oder verzweigtem (C₁-C₆)-Dialkylamino oder Halogenatomen substituiert sind,
- der Begriff «substituiert», bezogen auf die Begriffe «Cycloalkyl» und «Cycloalkylalkyl» bedeutet, daß der cyclische Teil dieser Gruppen durch eine oder mehrere gleichartige oder verschiedene Gruppen ausgewählt aus Hydroxy, geradkettigem oder verzweigtem (C₁-C₆)-Alkoxy, geradkettigem oder verzweigtem (C₁-C₆)-Polyhalogenalkyl, Amino, geradkettigem oder verzweigtem (C₁-C₆)-Alkylamino, geradkettigem oder verzweigtem (C₁-C₆)-Dialkylamino oder Halogenatomen substituiert ist,
deren Enantiomere und Diastereoisomere sowie deren Additionssalze mit einer pharmazeutisch annehmbaren Säure oder Base.

2. Verbindungen der Formel (I) nach Anspruch 1, worin Cy eine cyclische Struktur der Formel (II) bedeutet, deren Enantiomere und Diastereoisomere sowie deren Additionssalze mit einer pharmazeutisch annehmbaren Säure oder Base.

3. Verbindungen der Formel (I) nach Anspruch 1, worin Cy eine Naphthalingruppe bedeutet, deren Enantiomere und Diastereoisomere sowie deren Additionssalze mit einer pharmazeutisch annehmbaren Säure oder Base.

4. Verbindungen der Formel (I) nach Anspruch 1, worin Cy eine cyclische Struktur der Formel (III) bedeutet, deren Enantiomere und Diastereoisomere sowie deren Additionssalze mit einer pharmazeutisch annehmbaren Säure oder Base.

5. Verbindungen der Formel (I) nach Anspruch 1, worin Cy eine Benzothiophen- oder Benzofurangruppe bedeutet, deren Enantiomere und Diastereoisomere sowie deren Additionssalze mit einer pharmazeutisch annehmbaren Säure oder Base.

6. Verbindungen der Formel (I) nach Anspruch 1, worin Cy eine Indolgruppe bedeutet, deren Enantiomere und Diastereoisomere sowie deren Additionssalze mit einer pharmazeutisch annehmbaren Säure oder Base.

7. Verbindungen der Formel (I) nach Anspruch 1, worin Cy eine Azaindolgruppe bedeutet, deren Enantiomere und Diastereoisomere sowie deren Additionssalze mit einer pharmazeutisch annehmbaren Säure oder Base.

8. Verbindungen der Formel (I) nach Anspruch 1, worin G₂ eine Einfachbindung bedeutet, deren Enantiomere und Diastereoisomere sowie deren Additionssalze mit einer pharmazeutisch annehmbaren Säure oder Base.

9. Verbindungen der Formel (I) nach Anspruch 1, worin G₂ eine Gruppe -W₄-(CH₂)ₚ-W'₄- bedeutet, worin W₄ und W'₄, die gleichartig oder verschieden sind, ein Sauerstoffatom oder ein Schwefelatom oder eine Gruppe NH oder NRₐ und p eine ganze Zahl, die die Beziehung 1 ≤ p ≤ 12 erfüllt, bedeuten, deren Enantiomere und Diastereoisomere sowie deren Additionssalze mit einer pharmazeutisch annehmbaren Säure oder Base.

10. Verbindungen der Formel (I) nach Anspruch 1, worin G₂ eine Gruppe -O-(CH₂)ₚ-O- bedeutet, worin p eine ganze Zahl darstellt, so daß die Beziehung 1 ≤ p ≤ 12 erfüllt ist, deren Enantiomere und Diastereoisomere sowie deren Additionssalze mit einer pharmazeutisch annehmbaren Säure oder Base.

11. Verbindungen der Formel (I) nach Anspruch 1, worin G₂ eine Gruppe W₄-(CH₂)_{p'}-W'₄-(CH₂)_{p"} -W"₄- darstellt, worin W₄, W'₄ und W"₄, die gleichartig oder verschieden sind, ein Sauerstoffatom, ein Schwefelatom oder eine Gruppe NH oder NRₐ und p' und p" ganze Zahlen bedeuten, so daß die Beziehung 2 ≤ p' + p" ≤ 12 erfüllt ist, deren Enantiomere und Diastereoisomere sowie deren Additionssalze mit einer pharmazeutisch annehmbaren Säure oder Base.

12. Verbindungen der Formel (I) nach Anspruch 1, worin G₂ eine Gruppe -O-(CH₂)_{p'} -O-(CH₂)ₚ -O- darstellt (worin p' und p" die oben angegebenen Bedeutungen besitzen), deren Enantiomere und Diastereoisomere sowie deren Additionssalze mit einer pharmazeutisch annehmbaren Säure oder Base.

13. Verbindungen der Formel (I) nach Anspruch 1, worin A und B, die gleichartig oder verschieden sind, eine Gruppe NR¹COR² oder CONR²R³ bedeuten, deren Enantiomere und Diastereoisomere sowie deren Additionssalze mit einer pharmazeutisch annehmbaren Säure oder Base.

14. Verbindungen der Formel (I) nach Anspruch 1, nämlich *N*-(2-{7-[2-({8-[2-(Acetylamino)-ethyl]-2-naphthyl}-oxy)-ethoxy]-1-naphthyl}-ethyl)-acetamid, *N*-(2-{7-{3-({8-[2-(Acetylamino)-ethyl]-2-naphthyl}-oxy)-propoxy]-1-naphthyl}-ethyl)-acetamid, *N*-(2-{7-[4-({8-[2-(Acetylamino)-ethyl]-2-naphthyl}-oxy)-butoxy]-1-naphthyl}-ethyl)-acetamid, *N*-[2-(7-{[6-({8-[2-(Acetylamino)-ethyl]-2-naphthyl}-oxy)-hexyl]-oxy}-1-naphthyl)-ethyl]-acetamid, *N*-[2-(7-{[5-({8-[2-(Acetylamino)-ethyl]-2-naphthyl}-oxy)-pentyl]-oxy}-1-naphthyl)-ethyl]-acetamid, *N*-[2-(7-{[8-({8-[2-(Acetylamino)-ethyl]-2-naphthyl}-oxy)-octyl]-oxy}-1-naphthyl)-ethyl]-acetamid, *N*-(2-(7-{[10-({8-[2-(Acetylamino)-ethyl]-2-naphthyl}-oxy)-decyl]-oxy}-1-naphthyl)-ethyl]-acetamid und *N*-(2-{7-[4-({8-[2-(Acetylamino)-ethyl]-7-methoxy-2-naphthyl}-oxy)-butoxy]-2-methoxy-1-naphthyl}-ethyl)-acetamid sowie deren Additionssalze mit einer pharmazeutisch annehmbaren Säure oder Base.

15. Verbindung der Formel (I) nach Anspruch 1, nämlich *N*-[2-(7-{2-[2-({8-[2-(Acetylamino)-ethyl]-2-naphthyl}-oxy)-ethoxy]-ethoxy}-1-naphthyl)-ethyl]-acetamid sowie dessen Additionssalze mit einer pharmazeutisch annehmbaren Säure oder Base.

16. Verbindungen der Formel (I) nach Anspruch 1, nämlich *N*-(2-{7-[4-({8-[2-(2-Furoylamino)-ethyl]-2-naphthyl}-oxy)-butoxy]-1-naphthyl}-ethyl)-2-furamid, 2-Brom-*N*-[2-(7-{4-[(8-{2-[(bromacetyl)-amino]-ethyl}-2-naphthyl)-oxy]-butoxy}-1-naphthyl)-ethyl]-acetamid, *N*-(2-(7-{4-[(8-{2-[(Cyclopropylcarbonyl)-amino]-ethyl}-2-naphthyl)-oxy]-butoxy}-1-naphthyl)-ethyl]-cyclopropancarboxamid und *N*-(2-{7-[4-({8-[2-(3-Butenoylamino)-ethyl]-2-naphthyl}-oxy)-butoxy]-1-naphthyl}-ethyl)-3-butenamid sowie deren Additionssalze mit einer pharmazeutisch annehmbaren Säure oder Base.

17. Verbindung der Formel (I) nach Anspruch 1, nämlich *N*-(2-{7-[4-({8-[2-(Acetylamino)-ethyl]-5,6,7,8-tetrahydro-2-naphthalinyl}-oxy)-butoxy]-1,2,3,4-tetrahydro-1-naphthalinyl}-ethyl)-acetamid, dessen Enantiomere und Diastereoisomere sowie dessen Additionssalze mit einer pharmazeutisch annehmbaren Säure oder Base.

18. Verbindungen der Formel (I) nach Anspruch 1, nämlich *N*-{2-[5-(4-{[3-[2-(Acetylamino)-ethyl]-1-(phenylslfonyl)-*1H*-indol-5-yl]-oxy}-butoxy)-1-(phenylsulfonyl)-*1H*-indol-3-yl]-ethyl}-acetamid und *N*-(2-{5-[4-({3-[2-(Acetylamino)-ethyl]-*1H*-indol-5-yl}-oxy)-butoxy]-*1H*-indol-3-yl}-ethyl)-acetamid sowie deren Additionssalze mit einer pharmazeutisch annehmbaren Säure oder Base.

19. Verbindungen der Formel (I) nach Anspruch 1, nämlich *N*-(2-{5-[4-({3-[2-(Acetylamino)-ethyl]-1-benzofuran-5-yl}-oxy)-butoxy]-1-benzofuran-3-yl}-ethyl)-acetamid und *N*-(2-{5-[4-({3-[2-(Acetylamino)-ethyl]-1-benzothien-5-yl}-oxy)-butoxy]-1-benzothien-3-yl}-ethyl)-acetamid sowie deren Additionssalze mit einer pharmazeutisch annehmbaren Säure oder Base.

20. Verbindungen der Formel (I) nach Anspruch 1, nämlich *N*-(2-{5-[4-({3-[2-(Acetylamino)-ethyl]-1-methyl-*1H*-pyrrolo*[3,2-b]*pyridin-5-yl}-oxy)-butoxy]-1-methyl-*1H*-pyrrolo*[3,2-b]*pyridin-3-yl}-ethyl)-acetamid und *N*-(2-{5-[4-({3-[2-(Acetylamino)-ethyl]-1-methyl]-*1H*-pyrrolo*[3,2-b]*pyridin-5-yl}-oxy)-propoxy]-1-methyl-*1H*pyrrolo*[3,2-b]*pyridin-3-yl}-ethyl)-acetamid sowie deren Additionssalze mit einer pharmazeutisch annehmbaren Säure oder Base.

21. Verbindung der Formel (I) nach Anspruch 1, nämlich *N*-[2-(7-{8-[2-(Acetylamino)-ethyl]-2-naphthyl}-1-naphthyl)-ethyl]-acetamid sowie dessen Additionssalze mit einer pharmazeutisch annehmbaren Säure oder Base.

22. Verbindung der Formel (I) nach Anspruch 1, nämlich *N*-{2-[5-{3-[2-(Acetylamino)-ethyl]-*1H*-indol-3-yl}-*1H*-indol-3-yl]-ethyl}-acetamid sowie dessen Additionssalze mit einer pharmazeutisch annehmbaren Säure oder Base.

23. Verfahren zur Herstellung der Verbindungen der Formel (I) nach Anspruch 1, **dadurch gekennzeichnet, daß** als Ausgangsprodukt die Verbindung der Formel (V) verwendet:
A - G₁ - Cy - OMe (V)
in der A, G₁ und Cy die bezüglich der Formel (I) angegebenen Bedeutungen besitzen,
welche man einer Demethylierung unterwirft unter Verwendung von klassischen Mitteln, wie HBr, AlCl₃, AlBr₃, BBr₃ oder binäre Lewis-Säure/Nucleophil-Systeme, wie beispielsweise AlCl₃ / PhCH₂SH oder BBr₃ / Me₂S, zur Bildung der Verbindung der Formel (VI):
A - G₁ - Cy - OH (VI)
in der A, G₁ und Cy die oben angegebenen Bedeutungen besitzen,
◆ welche in klassischer Weise
- durch Einwirkung von beispielsweise *N,N*-Dimethylthiocarbamidsäure-Natriumsalz in das entsprechende Thiol der Formel (VII):
A - G₁ - Cy - SH (VII)
in der A, G₁ und Cy die oben angegebenen Bedeutungen besitzen,
- oder in das entsprechende Aminderivat der Formel (VIII) umgewandelt wird:
A - G₁ - Cy - NHR'ₐ (VIII)
in der A, G₁ und Cy die oben angegebenen Bedeutungen besitzen und R'ₐ sämtliche Bedeutungen für Rₐ annehmen kann, wie sie bezüglich der Formel (I) definiert worden sind, und zusätzlich ein Wasserstoffatom bedeuten kann,
wobei die Verbindungen der Formeln (VI), (VII) und (VIII) die Verbindung der Formel (IX) darstellen:
A-G₁-Cy-W₄H (IX)
worin W₄ ein Sauerstoffatom oder ein Schwefelatom oder eine Gruppe NH oder NRₐ (worin Rₐ die oben angegebenen Bedeutungen besitzt) darstellt, welche Verbindung der Formel (IX) man mit:
• einer Verbindung der Formel (X) kondensiert: 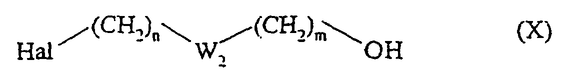 in der Hal ein Bromatom, ein Chloratom oder ein Iodatom bedeutet und n, W₂ und m die bezüglich der Formel (I) angegebenen Bedeutungen besitzen (mit der Maßgabe, daß nicht zwei aufeinanderfolgende Heteroatome vorliegen und die in dieser Weise definierte Kette eine oder mehrere Unsättigungen aufweisen kann),
• oder mit einer Verbindung der Formel (XI) kondensiert: 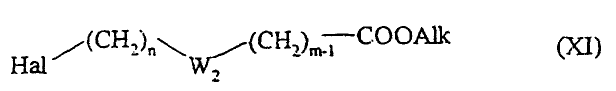 in der Hal, W₂, n und m die oben angegebenen Bedeutungen besitzen und Alk eine Alkylgruppe darstellt (wobei es sich versteht, daß nicht zwei aufeinanderfolgende Heteroatome vorliegen und daß die in dieser Weise definierte Kette eine oder mehrere Unsättigungen aufweisen kann), gefolgt von einer Reduktion,
so daß man die Verbindung der Formel (XII) erhält:
A - G₁ - Cy - W₄ - (CH₂)ₙ - W₂ - (CH₂)ₘ - OH (XII)
in der A, G₁, Cy, W₂, W₄, n und m die bezüglich der Formel (I) angegebenen Bedeutungen besitzen (wobei es sich versteht, daß nicht zwei aufeinanderfolgende Heteroatome in der Kette W₄-(CH₂)ₙ-W₂-(CH₂)ₘ-OH vorliegen und daß die in dieser Weise definierte Kette eine oder mehrere Unsättigungen aufweisen kann),
deren Hydroxylfunktion in klassischer Weise in eine austretende Gruppe, wie beispielsweise eine Mesylat-, eine Tosylatgruppe oder ein Halogenatom umgewandelt wird zur Bildung der Verbindung der Formel (XII'):
A - G₁ - Cy - W₄ - (CH₂)ₙ - W₂ - (CH₂)ₘ - E (XII')
in der A, G₁, Cy, W₄, n, W₂ und m die oben angegebenen Bedeutungen besitzen und E eine Mesyl- oder Tosylgruppe oder ein Halogenatom bedeutet, welche man mit einer Verbindung der Formel (XIII) umsetzt:
B - G₃ - Cy - W'₄H (XIII)
in der B, G₃ und Cy die bezüglich der Formel (I) angegebenen Bedeutungen besitzen und W'₄ die gleichen Bedeutungen besitzen kann wie die oben definierte Gruppe W₄,
zur Bildung der Verbindung der Formel (I/a), einem Sonderfall der Verbindungen der Formel (I):
A-G₁-Cy-W₄-(CH₂)ₙ-W₂-(CH₂)ₘ-W'₄-Cy-G₃-B (I/a)
in der A, G₁, Cy, W₄, n, W₂, m, W'₄, G₃ und B die oben angegebenen Bedeutungen besitzen,
(wobei die Verbindungen der Formel (I/a), worin die Gruppen A-G₁-Cy-W₄und W'₄-Cy-G₃-B identisch sind, direkt erhalten werden können ausgehend von der Verbindung der Formel (IX), welche man in basischem Medium mit einer Verbindung der Formel (X') kondensiert: 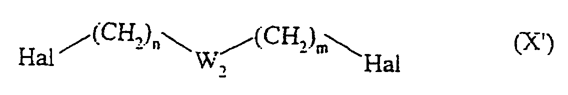 in der Hal, n, m und W₂ die oben angegebenen Bedeutungen besitzen),
◆ oder welche man unter Verwendung von beispielsweise Phenyl-bis(trifluormethan-sulfonimid) in basischem Medium in das entsprechende Trifluormethansulfonat der Formel (XIV) umwandelt:
A - G₁ - Cy - OSO₂CF₃ (XIV)
in der A, G₁ und Cy die oben angegebenen Bedeutungen besitzen,
- auf welches man unter den katalytischen Bedingungen eines geeigneten Palladiumderivats ein Borsäurederivat (R_{b}B(OH)₂) oder ein Zinnderivat (R_{b}SnBu₃) (worin R_{b} eine Gruppe der Formel (XV) darstellt:
B - G₃ - Cy - W₃ - (CH₂)ₘ - W₂ - (CH₂)ₙ-CH₂- (XV)
in der B, G₃, Cy, W₃, m, W₂ und n die oben angegebenen Bedeutungen besitzen, wobei es sich versteht, daß keine zwei aufeinanderfolgenden Heteroatome in der Kette -W₃-(CH₂)ₘ-W₂- vorliegen und daß die in dieser Weise definierte Kette eine oder mehrere Unsättigungen aufweisen kann) einwirken läßt,
zur Bildung der Verbindung der Formel (I/b), einem Sonderfall der Verbindungen der Formel (I):
A-G₁-Cy-CH₂-(CH₂)ₙ-W₂-(CH₂)ₘ-W₃-Cy-G₃-B (I/b)
in der A, G₁, Cy, n, W₂, m, W₃, Cy, G₃ und B die oben angegebenen Bedeutungen besitzen (wobei es sich versteht, daß nicht zwei aufeinanderfolgende Heteroatome in der Kette -W₂-(CH₂)ₘ-W₃- vorliegen und daß die in dieser Weise definierte Kette eine oder mehrere Unsättigungen aufweisen kann), wobei man die Verbindungen der Formel (I/c), einem Sonderfall der Verbindungen der Formel (I):
A-G₁-Cy-W₁-(CH₂)ₙ-W₂-(CH₂)ₘ-CH₂-Cy-G₃-B (I/c)
in der A, G₁, Cy, W₁, n, W₂, m, G₃ und B die oben angegebenen Bedeutungen besitzen (wobei es sich versteht, daß nicht zwei aufeinanderfolgende Heteroatome in der Kette -W₁-(CH₂)ₙ-W₂- vorliegen und daß die in dieser Weise definierte Kette eine oder mehrere Unsättigungen aufweisen kann), mit Hilfe einer ähnlichen Verfahrensweise ausgehend von der Verbindung der Formel (XIV') erhält:
B - G₃ - Cy - OSO₂CF₃ (XIV')
in der B, G₃ und Cy die obe angegebenen Bedeutungen besitzen,
- oder welche man unter Kupplungsbedingungen unter Verwendung von beispielsweise Nickel- oder Palladium-Verbindungen mit einer Verbindung der Formel (XIV') in Kontakt bringt, zur Bildung der Verbindung der Formel (I/d), einem Sonderfall der Verbindungen der Formel (I):
A-G₁-Cy-Cy-G₃-B (I/d)
in der A, G₁, Cy, G₃ und B die oben angegebenen Bedeutungen besitzen,
wobei die Gesamtheit der Verbindungen (I/a) bis (I/d) die Verbindung der Formel (I) bilden, welche gewünschtenfalls mit Hilfe einer klassischen Reinigungsmethode gereinigt werden kann, welche man gegebenenfalls mit Hilfe einer klassischen Trennmethode in ihre Isomeren auftrennen kann und welche man gegebenenfalls in ihre Additionssalze mit einer pharmazeutisch annehmbaren Säure oder Base umwandeln kann.

24. Verbindungen der Formel (XII"):
A - G₁ - Cy - W₄ - (CH₂)ₙ - W₂ - (CH₂)ₘ - E' (XIIʺ)
in der:
◆ A eine Gruppe der Formel 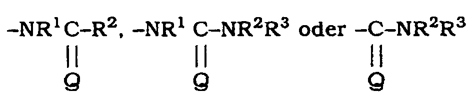 darstellt, in denen:
- Q ein Schwefelatom oder ein Sauerstoffatom darstellt,
- R¹, R² und R³, die gleichartig oder verschieden sind, ein Wasserstoffatom oder eine Gruppe Rₐ bedeuten [worin Rₐ eine geradkettige oder verzweigte, nichtsubstituierte oder substituierte (C₁-C₆)-Alkylgruppe, geradkettige oder verzweigte, nichtsubstituierte oder substituierte (C₂-C₆)-Alkenylgruppe, geradkettige oder verzweigte, nichtsubstituierte oder substituierte (C₂-C₆)-Alkinylgruppe, nichtsubstituierte oder substituierte (C₃-C₈)-Cycloalkylgruppe, geradkettige oder verzweigte, nichtsubstituierte oder substituierte (C₃-C₈)-Cycloalkyl-(C₁-C₆)-alkylgruppe, geradkettige oder verzweigte (C₁-C₆)-Polyhalogenalkylgruppe, Arylgruppe, Arylalkylgruppe (in der der Alkylrest 1 bis 6 Kohlenstoffatome enthält und geradkettig oder verzweigt sein kann), Arylalkenylgruppe (worin der Alkenylrest 2 bis 6 Kohlenstoffatome enthält und geradkettig oder verzweigt sein kann), Heteroarylgruppe, Heteroarylalkylgruppe (worin der Alkylrest 1 bis 6 Kohlenstoffatome enthält und geradkettig oder verzweigt sein kann) oder Heteroarylalkenylgruppe (worin der Alkenylrest 2 bis 6 Kohlenstoffatome enthält und geradkettig oder verzweigt sein kann) bedeutet],
oder die Gruppen R² und R³ zusammen mit dem sie tragenden Stickstoffatom eine Gruppe ausgewählt aus Piperazinyl, Piperidinyl oder Pyrrolidinyl bilden,
◆ G₁ eine geradkettige oder verzweigte Alkylenkette bedeutet, die 1 bis 4 Kohlenstoffatome enthält und gegebenenfalls durch eine oder mehrere gleichartige oder verschiedene Gruppen ausgewählt aus Hydroxy, Carboxy, Formyl, Rₐ, ORₐ, COORₐ oder CORₐ (worin Rₐ die oben angegebenen Bedeutungen besitzt) substituiert ist,
◆ Cy
- eine cyclische Struktur der Formel (II): 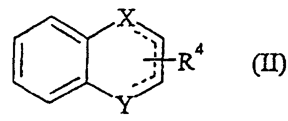 in der:
∗ X und Y, die gleichartig oder verschieden sind, ein Schwefelatom, ein Sauerstoffatom oder ein Kohlenstoffatom oder eine Gruppe CH oder CH₂ bedeuten,
∗ R⁴ ein Wasserstoffatom, ein Halogenatom oder eine CF₃-Gruppe, Hydroxygruppe, Carboxygruppe, Formylgruppe, Aminogruppe, eine Gruppe der Formel NHRₐ, NRₐR¹ₐ, NHCORₐ, CONHRₐ, Rₐ, ORₐ, CORₐ oder COORₐ darstellt (worin Rₐ die oben angegebenen Bedeutungen besitzt und R¹ₐ sämtliche Bedeutungen von Rₐ annehmen kann),
∗ das Symbol ---- bedeutet, daß die Bindungen einfach oder doppelt sind, wobei es sich versteht, daß die Wertigkei der Atome respektiert ist,
worin G₁ den X und Y enthaltenden Ring substituiert,
- oder eine cyclische Struktur der Formel (III) bedeutet: 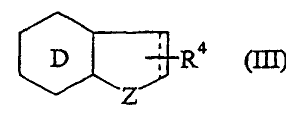 in der:
∗ Z ein Schwefelatom, ein Sauerstoffatom oder eine Gruppe CH, CH₂, NH, NSO₂Pb oder NRₐ darstellt (worin Rₐ die oben angegebenen Bedeutungen besitzt), darstellt,
∗ D einen Benzol- oder Pyridin-Ring darstellt,
∗ R⁴ die oben angegebenen Bedeutungen besitzt,
∗ das Symbol ---- bedeutet, daß die Bindung einfach oder doppelt ist, wobei es sich versteht, daß die Wertigkeit der Atome respektiert wird,
worin G₁ den Z enthaltenden Ring substituiert,
◆ W₂ eine Bindung, ein Sauerstoffatom, ein Schwefelatom oder eine Gruppe CH₂, CHRₐ, NH oder NRₐ darstellt (worin Rₐ die oben angegebenen Bedeutungen besitzt),
◆ W₄ ein Sauerstoffatom, ein Schwefelatom oder eine Grppe NH oder NRₐ darstellt (worin Rₐ die oben angegebenen Bedeutungen besitzt).
◆ n eine ganze Zahl darstellt, derart, daß die Beziehung 0 ≤ n ≤ 6 erfüllt ist,
◆ m eine ganze Zahl darstellt, derart, daß die Beziehung 0 ≤ m ≤ 6 erfüllt ist, mit der Maßgabe, daß nicht zwei aufeinanderfolgende Heteroatome vorliegen und daß die in dieser Weise definierte Kette -W₄-(CH₂)ₙ-W₂-(CH₂)ₘ- eine oder mehrere Unsättigungen aufweisen kann,
◆ und E' eine Hydroxylgruppe oder ein Halogenatom (Fluor, Chlor, Brom oder Iod) bedeutet,
mit der Maßgabe, daß
- wenn Cy eine Naphthalingruppe und G₁-A eine Gruppe -(CH₂)₂-NR¹C(Q)R² oder - (CH₂)₂-NR¹C(Q)NR²R³ (worin R¹, R² und R³ die oben angegebenen Bedeutungen besitzen) darstellen, dann die Kette -W₄-(CH₂)ₙ-W₂-(CH₂)ₘ- keine -O-Alkylkette darstellen kann,
- die Verbindung der Formel (XII") nicht *N*-{2-[5-(2-Hydroxyethoxy)-*1H*-indol-3-yl]-ethyl}-acetamid bedeutet,
deren Enantiomere und Diastereoisomere sowie deren Additionssalze mit einer pharmazeutisch annehmbaren Säure oder Base,
als Synthesezwischenprodukte und auch als Verbindung, die zur Behandlung von Störungen des melatoninergischen Systems nützlich sind.

25. Pharmazeutische Zubereitungen enthaltend als Wirkstoff mindestens eine Verbindung der Formel (I) nach irgendeinem der Ansprüche 1 bis 22 oder 24 oder eines ihrer Additionssalze mit einer pharmazeutisch annehmbaren Säure oder Base in Kombination mit einem oder mehreren pharmazeutisch annehmbaren Trägermaterialien.

26. Pharmazeutische Zubereitungen nach Anspruch 25 nützlich für die Herstellung eines Arzneimittels zur Behandlung von Störungen, die mit dem melatoninergischen System verknüpft sind.

## Claims

1. Compounds of formula (I):
A-G₁-Cy-G₂-Cy-G₃-B (I)
wherein:
◆ A represents a grouping of formula 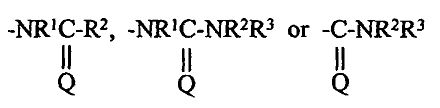 wherein:
- Q represents a sulphur or oxygen atom,
- R¹, R² and R³, which may be identical or different, represent a hydrogen atom or a group Rₐ [wherein Rₐ represents an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, an unsubstituted or substituted (C₃-C₈)-cycloalkyl group, an unsubstituted or substituted (C₃-C₈)cycloalkyl-(C₁-C₆)alkyl group in which the alkyl moiety is linear or branched, a polyhalo-(C₁-C₆)alkyl group in which the alkyl moiety is linear or branched, an aryl group, an arylalkyl group (in which the alkyl moiety contains from 1 to 6 carbon atoms and may be linear or branched), an arylalkenyl group (in which the alkenyl moiety contains from 2 to 6 carbon atoms and may be linear or branched), a heteroaryl group, a heteroarylalkyl group (in which the alkyl moiety contains from 1 to 6 carbon atoms and may be linear or branched) or a heteroarylalkenyl group (in which the alkenyl moiety contains from 2 to 6 carbon atoms and may be linear or branched)],
or the groupings R² and R³ form, with the nitrogen atom carrying them, a group selected from piperazinyl, piperidinyl and pyrrolidinyl,
◆ B represents a grouping of formula 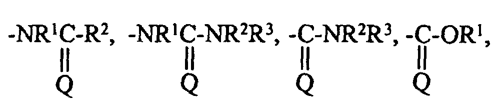 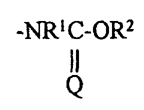 or -NR²R³ wherein Q, R¹, R² and R³ are as defined hereinbefore,
◆ G₁ and G₃, which may be identical or different, represent a linear or branched alkylene chain having from 1 to 4 carbon atoms that is optionally substituted by one or more identical or different groups selected from hydroxy, carboxy, formyl, Rₐ, ORₐ, COORₐ and CORₐ (wherein Rₐ is as defined hereinbefore),
◆ Cy represents
- a ring structure of formula (II) : 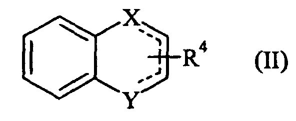 wherein:
∗ X and Y, which may be identical or different, represent a sulphur, oxygen or carbon atom, or a CH or CH₂ group,
∗ R⁴ represents a hydrogen or halogen atom, or a CF₃, hydroxy, carboxy, formyl, amino, NHRₐ, NRₐR¹ₐ, NHCORₐ, CONHRₐ, Rₐ, ORₐ, CORₐ or COORₐ group (wherein Rₐ is as defined hereinbefore and R¹ₐ can have any of the meanings of Rₐ),
∗ the symbol ---- means that the bonds are single or double, with the proviso that the valency of the atoms is respected,
wherein G₂ substitutes the benzene ring, and G₁ (and G₃ respectively) substitutes the ring containing X and Y,
- or a ring structure of formula (III) : 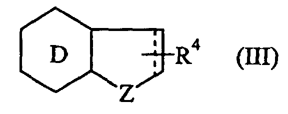 wherein:
∗ Z represents a sulphur or oxygen atom, or a CH, CH₂, NH, NSO₂Ph or NRₐ group (wherein Rₐ is as defined hereinbefore),
∗ D represents a benzene or pyridine ring,
∗ R⁴ is as defined hereinbefore,
∗ the symbol ---- means that the bond is single or double, with the proviso that the valency of the atoms is respected,
wherein G₂ substitutes the D ring, and G₁ (and G₃ respectively) substitutes the ring containing Z,
it being understood that the two rings (Cy) of the compounds of formula (I) represent the same basic ring structure (indole/indole, naphthalene/naphthalene, benzofuran/ benzofuran, etc.), but the substituent R⁴ may be different,
◆ G₂ represents a chain of formula (IV): 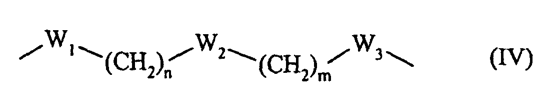 wherein:
- W₁, W₂ and W₃, which may be identical or different, represent a bond, an oxygen or sulphur atom, or a CH₂, CHRₐ, NH or NRₐ group (wherein Rₐ is as defined hereinbefore),
- n represents an integer such that 0 ≤ n ≤ 6,
- m represents an integer such that 0 ≤ m ≤ 6,
with the proviso that it is not possible to have two consecutive hetero atoms and that the chain of formula (IV) so defined may have one or more unsaturations,
wherein :
- the compound of formula (I) cannnot represent diethyl 2-(acetylamino)-2-{[5-({3-[2-(acetylamino)-3-ethoxy-2-(ethoxycarbonyl)-3-oxopropyl]-*1H*-indol-5-yl}methyl}-*1H*-indol-3-yl]methyl}malonate, diethyl 2-(acetylamino)-2-{[5-{3[-[2-(acetylamino)-3-ethoxy-2-(ethoxycarbonyl)-3-oxopropyl]-2-(ethoxycarbonyl)-1*H*-indol-5-yl]methyl}-2-(ethoxycarbonyl)-*1H*-indol-3-yl}methyl)-malonate, or N-{2-[5-({3-[2-(acetylamino)ethyl]-*1H*-indol-5-yl}methyl)-*1H*-indol-3-yl]-ethyl}acetamide,
- "aryl" is understood to mean the groups naphthyl, phenyl and biphenyl,
- "heteroaryl" is understood to mean any saturated or unsaturated mono- or bicyclic group containing from 5 to 10 atoms and containing from 1 to 3 hetero atoms selected from nitrogen, sulphur and oxygen, it being possible for the "aryl" and "heteroaryl" groups to be substituted by one or more identical or different radicals selected from hydroxy, carboxy, linear or branched (C₁-C₆)alkoxy, linear or branched (C₁-C₆)alkyl, polyhalo-(C₁-C₆)-alkyl in which the alkyl moiety is linear or branched, formyl, cyano, nitro, amino, linear or branched (C₁-C₆)alkylamino, di-(C₁-C₆)alkylamino in which each alkyl moiety is linear or branched, and halogen atoms,
- the term "substituted" applied to the terms "alkyl", "alkenyl" and "alkynyl" means that those groups are substituted by one or more identical or different radicals selected from hydroxy, linear or branched (C₁-C₆)alkoxy, polyhalo-(C₁-C₆)alkyl in which the alkyl moiety is linear or branched, amino, linear or branched (C₁-C₆)alkylamino, di-(C₁-C₆)alkylamino in which each alkyl moiety is linear or branched, and halogen atoms,
- the term "substituted" applied to the terms "cycloalkyl" and "cycloalkylalkyl" means that the cyclic moiety of those groups is substituted by one or more identical or different radicals selected from hydroxy, linear or branched (C₁-C₆)alkoxy, polyhalo-(C₁-C₆)alkyl in which the alkyl moiety is linear or branched, amino, linear or branched (C₁-C₆)alkylamino, di-(C₁-C₆)alkylamino in which each alkyl moiety is linear or branched, and halogen atoms,
their enantiomers and diastereoisomers, and addition salts thereof with a pharmaceutically acceptable acid or base.

2. Compounds of formula (I) according to claim 1 wherein Cy represents a ring structure of formula (II), their enantiomers and diastereoisomers, and addition salts thereof with a pharmaceutically acceptable acid or base.

3. Compounds of formula (I) according to claim 1 wherein Cy represents a naphthalene, their enantiomers and diastereoisomers, and addition salts thereof with a pharmaceutically acceptable acid or base.

4. Compounds of formula (I) according to claim 1 wherein Cy represents a ring structure of formula (III), their enantiomers and diastereoisomers, and addition salts thereof with a pharmaceutically acceptable acid or base.

5. Compounds of formula (I) according to claim 1 wherein Cy represents a benzothiophene or a benzofuran, their enantiomers and diastereoisomers, and addition salts thereof with a pharmaceutically acceptable acid or base.

6. Compounds of formula (I) according to claim 1 wherein Cy represents an indole, their enantiomers and diastereoisomers, and addition salts thereof with a pharmaceutically acceptable acid or base.

7. Compounds of formula (I) according to claim 1 wherein Cy represents an azaindole, their enantiomers and diastereoisomers, and addition salts thereof with a pharmaceutically acceptable acid or base.

8. Compounds of formula (I) according to claim 1 wherein G₂ represents a single bond, their enantiomers and diastereoisomers, and addition salts thereof with a pharmaceutically acceptable acid or base.

9. Compounds of formula (I) according to claim 1 wherein G₂ represents a grouping -W₄-(CH₂)ₚ-W'₄- wherein W₄ and W'₄, which may be identical or different, represent an oxygen or sulphur atom or an NH or NRₐ group, and p represents an integer such that 1 ≤ p ≤ 12, their enantiomers and diastereoisomers, and addition salts thereof with a pharmaceutically acceptable acid or base.

10. Compounds of formula (I) according to claim 1 wherein G₂ represents a grouping -O-(CH₂)ₚ-O- wherein p represents an integer such that 1 ≤ p ≤ 12, their enantiomers and diastereoisomers, and addition salts thereof with a pharmaceutically acceptable acid or base.

11. Compounds of formula (I) according to claim 1 wherein G₂ represents a grouping W₄-(CH₂)_{p'}-W'₄-(CH₂)_{p"}-W"₄- wherein W₄, W'₄ and W"₄, which may be identical or different, represent an oxygen or sulphur atom or an NH or NRₐ group, and p' and p" are integers such that 2 ≤ p' + p" ≤ 12, their enantiomers and diastereoisomers, and addition salts thereof with a pharmaceutically acceptable acid or base.

12. Compounds of formula (I) according to claim 1 wherein G₂ represents a grouping -O-(CH₂)_{p'}-O-(CH₂)_{p"}-O- (wherein p' and p" are as defined hereinbefore), their enantiomers and diastereoisomers, and addition salts thereof with a pharmaceutically acceptable acid or base.

13. Compounds of formula (I) according to claim 1 wherein A and B, which may be identical or different, represent a grouping NR¹COR² or CONR²R³, their enantiomers and diastereoisomers, and addition salts thereof with a pharmaceutically acceptable acid or base.

14. Compounds of formula (I) according to claim 1 which are *N*-(2-{7-[2-({8-[2-(acetylamino)ethyl]-2-naphthyl}oxy)ethoxy]-1-naphthyl}ethyl)acetamide, *N*-(2-{7-[3-({8-[2-(acetylamino)ethyl]-2-naphthyl}oxy)propoxy]-1-naphthyl}ethyl)acetamide, *N*-(2-{7-[4-({8-[2-(acetylamino)ethyl]-2-naphthyl}oxy)butoxy]-1-naphthyl}ethyl)acetamide, *N*-[2-(7-{[6-({8-[2-(acetylamino)ethyl]-2-naphthyl}oxy)hexyl]oxy}-1-naphthyl)ethyl]acetamide, *N*-[2-(7-{[5-({8-[2-(acetylamino)ethyl]-2-naphthyl}oxy)pentyl]oxy}-1-naphthyl)ethyl]-acetamide, *N*-[2-(7-{[8-({8-[2-(acetylamino)ethyl]-2-naphthyl}oxy)octyl]oxy}-1-naphthyl)ethyl]acetamide, *N*-[2-(7-{[10-({8-[2-(acetylamino)ethyl]-2-naphthyl}oxy)-decyl]oxy}-1-naphthyl)ethyl]acetamide and *N*-(2-{7-[4-({8-[2-(acetylamino)ethyl]-7-methoxy-2-naphthyl}oxy)butoxy]-2-methoxy-1-naphthyl}ethyl)acetamide, and addition salts thereof with a pharmaceutically acceptable acid or base.

15. Compound of formula (I) according to claim 1 which is *N*-[2-(7-{2-[2-({8-[2-(acetylamino)ethyl]-2-naphthyl}oxy)ethoxy]ethoxy}-1-naphthyl)ethyl]acetamide and its addition salts with a pharmaceutically acceptable acid or base.

16. Compounds of formula (I) according to claim 1 which are *N*-(2-{7-[4-({8-[2-(2-furoylamino)ethyl]-2-naphthyl}oxy}butoxy]-1-naphthyl}ethyl)-2-furamide, 2-bromo-*N*-[2-(7-{4-[(8-{2-[(bromoacetyl)amino]ethyl}-2-naphthyl)oxy]butoxy}-1-naphthyl)ethyl]-acetamide, *N*-[2-(7-{4-[(8-{2-[(cyclopropylcarbonyl)amino]ethyl}-2-naphthyl)oxy]butoxy}-1-naphthyl)ethyl]cyclopropanecarboxamide and *N*-(2-{7-[4-({8-[2-(3-butenoylamino)ethyl]-2-naphthyl}oxy)butoxy]-1-naphthyl}ethyl)-3-butenamide, and addition salts thereof with a pharmaceutically acceptable acid or base.

17. Compound of formula (I) according to claim 1 which is *N*-(2-{7-[4-({8-[2-(acetylamino)ethyl]-5,6,7,8-tetrahydro-2-naphthalenyl}oxy)butoxy]-1,2,3,4-tetrahydro-1-naphthalenyl}ethyl)acetamide, its enantiomers and diastereoisomers, and addition salts thereof with a pharmaceutically acceptable acid or base.

18. Compounds of formula (I) according to claim 1 which are *N*-{2-[5-(4-{[3-[2-(acetylamino)ethyl]-1-(phenylsulphonyl)-*1H*-indol-5-yl]oxy}butoxy)-1-(phenylsulphonyl)-*1H*-indol-3-yl]ethyl}acetamide and *N*-(2-{5-[4-({3-[2-(acetylamino)ethyl]-*1H*-indol-5-yl}oxy)butoxy]-*1H*-indol-3-yl}ethyl)acetamide, and addition salts thereof with a pharmaceutically acceptable acid or base.

19. Compounds of formula (I) according to claim 1 which are *N*-(2-{5-[4-({3-[2-(acetylamino)ethyl]-1-benzofuran-5-yl}oxy)butoxy]-1-benzofuran-3-yl}ethyl)acetamide and *N*-(2-{5-[4-({3-[2-(acetylamino)ethyl]-1-benzothien-5-yl}oxy)butoxy]-1-benzothien-3-yl}ethyl)acetamide, and addition salts thereof with a pharmaceutically acceptable acid or base.

20. Compounds of formula (I) according to claim 1 which are *N*-(2-{5-[4-({3-[2-(acetylamino)ethyl]-1-methyl-*1H*-pyrrolo*[3,2-b]*pyridin-5-yl}oxy)butoxy]-1-methyl-*1H*-pyrrolo*[3,2-b]*pyridin-3-yl}ethyl)acetamide and *N*-(2-{5-[4-({3-[2-(acetylamino)ethyl]-1-methyl-*1H*-pyrrolo*[3,2-b]*pyridin-5-yl}oxy)propoxy]-1-methyl-*1H*-pyrrolo*[3,2-b]*pyridin-3-yl}ethyl)acetamide, and addition salts thereof with a pharmaceutically acceptable acid or base.

21. Compound of formula (I) according to claim 1 which is *N*-[2-(7-{8-[2-(acetylamino)ethyl]-2-naphthyl}-1-naphthyl)ethyl]acetamide, and addition salts thereof with a pharmaceutically acceptable acid or base.

22. Compound of formula (I) according to claim 1 which is *N*-{2-[5-{3-[2-(acetylamino)ethyl]-*1H*-indol-3-yl}-*1H*-indol-3-yl]ethyl}acetamide, and addition salts thereof with a pharmaceutically acceptable acid or base.

23. Process for the preparation of compounds of formula (I) according to claim 1, **characterised in that** there is used as starting material a compound of formula (V):
A-G₁-Cy-OMe (V)
wherein A, G₁ and Cy are as defined for formula (I),
which is subjected to demethylation using conventional agents, such as HBr, AlCl₃, AlBr₃, BBr₃ or Lewis acid/nucleophile binary systems, such as, for example, AlCl₃ / PhCH₂SH or BBr₃ / Me₂S, to obtain a compound of formula (VI):
A-G₁-Cy-OH (VI)
wherein A, G₁ and Cy are as defined hereinbefore,
◆ which is converted, in conventional manner,
- by the action of, for example, sodium *N,N*-dimethylthiocarbamate, to the corresponding thiol of formula (VII):
A-G₁-Cy-SH (VII)
wherein A, G₁ and Cy are as defined hereinbefore,
- or to the corresponding amine compound of formula (VIII)
A-G₁-Cy-NHR'ₐ (VIII)
wherein A, G₁ and Cy are as defined hereinbefore and R'ₐ can have any of the meanings of Rₐ as defined for formula (I) and can also represent a hydrogen atom,
which compounds of formulae (VI), (VII) and (VIII) represent the compound of formula (IX) :
A-G₁-Cy-W₄H (IX)
wherein W₄ represents an oxygen or sulphur atom, or an NH or NRₐ group (wherein Rₐ is as defined hereinbefore),
which compound of formula (IX) is condensed with :
• a compound of formula (X): 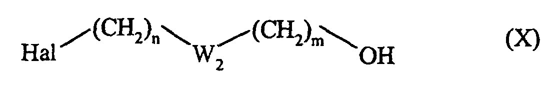 wherein Hal represent a bromine, chlorine or iodine atom, and n, W₂ and m are as defined for formula (I), (with the proviso that it is not possible to have two consecutive hetero atoms and that the chain so defined may have one or more unsaturations),
• or a compound of formula (XI) : 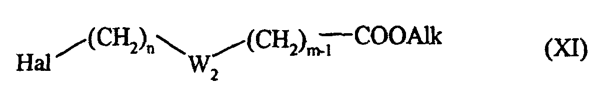 wherein Hal, W₂, n and m are as defined hereinbefore and Alk represents an alkyl radical (with the proviso that it is not possible to have two consecutive hetero atoms and that the chain so defined may have one or more unsaturations), followed by reduction,
to yield a compound of formula (XII) :
A-G₁-Cy-W₄-(CH₂)ₙ-W₂-(CH₂)ₘ-OH (XII)
wherein A, G₁, Cy, W₄, n and m are as defined for formula (I) (with the proviso that it is not possible to have two consecutive hetero atoms in the W₄-(CH₂)ₙ-W₂-(CH₂)ₘ-OH chain and that the chain so defined may have one or more unsaturations),
the hydroxyl function of which is converted in conventional manner to a leaving group, such as, for example, a mesylate, a tosylate, or a halogen compound, to yield a compound of formula (XII') :
A-G₁-Cy-W₄-(CH₂)ₙ-W₂-(CH₂)ₘ-E (XII')
wherein A, G₁, Cy, W₄, n, W₂ and m are as defined hereinbefore and E represents a mesyl or tosyl group or a halogen atom,
which is subjected to the action of a compound of formula (XIII) :
B-G₃-Cy-W'₄H (XIII)
wherein B, G₃ and Cy are as defined for formula (I) and W'₄ can have the same meanings as W₄ defined hereinbefore,
to yield a compound of formula (I/a), a particular case of the compounds of formula (I) :
A-G₁-Cy-W₄-(CH₂)ₙ-W₂-(CH₂)ₘ-W'₄-Cy-G₃-B (I/a)
wherein A, G₁, Cy, W₄, n, W₂, m, W'₄, G₃ and B are as defined hereinbefore,
(which compounds of formula (I/a) wherein the groupings A-G₁ -Cy-W₄- and W'₄-Cy-G₃-B are identical can be obtained directly from a compound of formula (IX) which is condensed, in a basic medium, with a compound of formula (X') : 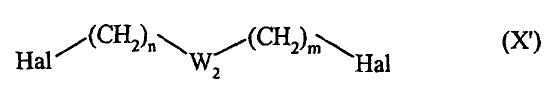 wherein Hal, n, m and W₂ are as defined hereinbefore),
◆ or converted using, for example, phenyl bis(trifluoromethanesulphonimide) in a basic medium to the corresponding trifluoromethanesulphonate of formula (XIV) :
A-G₁-Cy-OSO₂CF₃ (XIV)
wherein A, G₁ and Cy are as defined hereinbefore,
- which is subjected, under conditions of catalysis by a suitable palladium compound, to the action of a boric acid compound (R_{b}B(OH)₂) or of a tin compound (R_{b}SnBu₃) (wherein R_{b} represents a grouping of formula (XV) :
B-G₃-Cy-W₃-(CH₂)ₘ-W₂-(CH₂)ₙ-CH₂- (XV)
wherein B, G₃, Cy, W₃, m, W₂ and n are as defined hereinbefore, with the proviso that it is not possible to have two consecutive hetero atoms in the -W₃-(CH₂)ₘ-W₂- chain and that the chain so defined may have one or more unsaturations),
to yield a compound of formula (I/b), a particular case of the compounds of formula (I):
A-G₁-Cy-CH₂-(CH₂)ₙ-W₂-(CH₂)ₘ-W₃-Cy-G₃-B (I/b)
wherein A, G₁, Cy, n, W₂, m, W₃, Cy, G₃ and B are as defined hereinbefore (with the proviso that it is not possible to have two consecutive hetero atoms in the -W₂-(CH₂)ₘ-W₃ chain and that the chain so defined may have one or more unsaturations),
which compounds of formula (I/c), a particular case of the compounds of formula (I):
A-G₁-Cy-W₁-(CH₂)ₙ-W₂-(CH₂)ₘ-CH₂-G₃-B (I/c)
wherein A, G₁, Cy, W₁, n, W₂, m, G₃ and B are as defined hereinbefore (with the proviso that it is not possible to have two consecutive hetero atoms in the -W₁-(CH₂)ₙ-W₂- chain and that the chain so defined may have one or more unsaturations),
are obtained according to a similar procedure starting from a compound of formula (XIV') :
B-G₃-Cy-OSO₂CF₃ (XIV')
wherein B, G₃ and Cy are as defined hereinbefore,
- or is treated, under coupling conditions using, for example, nickel or palladium compounds, with a compound of formula (XIV') to yield a compound of formula (I/d), a particular case of the compounds of formula (I) :
A-G₁-Cy-Cy-G₃-B (I/d)
wherein A, G₁, Cy, G₃ and B are as defined hereinbefore,
the totality of the compounds (I/a) to (I/d) constituting the compound of formula (I) which may be purified, if desired, by a conventional purification technique, are optionally separated into their isomers according to a conventional separation technique, and converted, if necessary, into addition salts with a pharmaceutically acceptable acid or base.

24. Compounds of formula (XII"):
A-G₁-Cy-W₄-(CH₂)ₙ-W₂-(CH₂)ₘ,-E' (XIIʺ)
wherein :
◆ A represents a grouping of formula 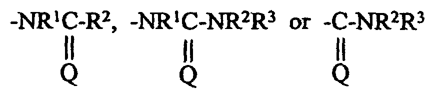 wherein:
- Q represents a sulphur or oxygen atom,
- R¹, R² and R³, which may be identical or different, represent a hydrogen atom or a group Rₐ [wherein Rₐ represents an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, an unsubstituted or substituted (C₃-C₈)-cycloalkyl group, an unsubstituted or substituted (C₃-C₈)cycloalkyl-(C₁-C₆)alkyl group in which the alkyl moiety is linear or branched, a polyhalo-(C₁-C₆)alkyl group in which the alkyl moiety is linear or branched, an aryl group, an arylalkyl group (in which the alkyl moiety contains from 1 to 6 carbon atoms and may be linear or branched), an arylalkenyl group (in which the alkenyl moiety contains from 2 to 6 carbon atoms and may be linear or branched), a heteroaryl group, a heteroarylalkyl group (in which the alkyl moiety contains from 1 to 6 carbon atoms and may be linear or branched) or a heteroarylalkenyl group (in which the alkenyl moiety contains from 2 to 6 carbon atoms and may be linear or branched)],
or the groupings R² and R³ form, with the nitrogen atom carrying them, a group selected from piperazinyl, piperidinyl and pyrrolidinyl,
◆ G₁ represents a linear or branched alkylene chain having from 1 to 4 carbon atoms that is optionally substituted by one or more identical or different groups selected from hydroxy, carboxy, formyl, Rₐ, ORₐ, COORₐ and CORₐ (wherein Rₐ is as defined hereinbefore),
◆ Cy represents
- a ring structure of formula (II): 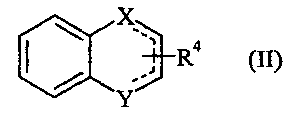 wherein:
∗ X and Y, which may be identical or different, represent a sulphur, oxygen or carbon atom, or a CH or CH₂ group,
∗ R⁴ represents a hydrogen or halogen atom, or a CF₃, hydroxy, carboxy, formyl, amino, NHRₐ, NRₐR¹ₐ, NHCORₐ, CONHRₐ, Rₐ, ORₐ, CORₐ or COORₐ group (wherein Rₐ is as defined hereinbefore and R¹ₐ can have any of the meanings of Rₐ),
∗ the symbol ---- means that the bonds are single or double, with the proviso that the valency of the atoms is respected,
wherein G₁ substitutes the ring containing X and Y,
- or a ring structure of formula (III): 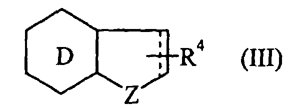 wherein:
∗ Z represents a sulphur or oxygen atom, or a CH, CH₂, NH, NSO₂Ph or NRₐ group (wherein Rₐ is as defined hereinbefore),
∗ D represents a benzene or pyridine ring,
∗ R⁴ is as defined hereinbefore,
∗ the symbol ---- means that the bond is single or double, with the proviso that the valency of the atoms is respected,
wherein G₁ substitutes the ring containing Z,
◆ W₂ represents a bond, an oxygen or sulphur atom, or a CH₂, CHRₐ, NH or NRₐ group (wherein Rₐ is as defined hereinbefore),
◆ W₄ represents an oxygen or sulphur atom or a NH or NRₐ group (wherein Rₐ is as defined hereinbefore),
◆ n an integer such that 0 ≤ n ≤ 6,
◆ m represents an integer such that 0 ≤ m **≤** 6,
with the proviso that it is not possible to have two consecutive hetero atoms, and that the -W₄-(CH₂)ₙ-W₂-(CH₂)ₘ- chain so defined may have one or more unsaturations,
◆ and E' represents a hydroxyl group or a halogen atom (fluorine, chlorine, bromine or iodine),
with the proviso that
- when Cy represents a naphthalene and G₁-A represents a grouping -(CH₂)₂-NR¹C(Q)R² or -(CH₂)₂-NR¹C(Q)NR²R³ (wherein R¹, R² and R³ are as defined hereinbefore), then the -W₄-(CH₂)ₙ-W₂-(CH₂)ₘ- chain cannot represent an -O-alkyl-chain,
- the compound of formula (XII") cannot represent *N*-{2-[5-(2-hydroxyethoxy)-*1H*-indol-3-yl]ethyl}acetamide,
their enantiomers and diastereoisomers, and addition salts thereof with a pharmaceutically acceptable acid or base,
as synthesis intermediates but also as compounds for use in the treatment of disorders associated with the melatoninergic system.

25. Pharmaceutical compositions comprising as active ingredient at least one compound of formula (I) according to any one of claims 1 to 22 or 24 or a pharmaceutically acceptable addition salt thereof with an acid or base in combination with one or more pharmaceutically acceptable excipients.

26. Pharmaceutical compositions according to claim 25 for use in the production of a medicament to treat disorders associated with the melatoninergic system.
